# EUROPEAN PATENT SPECIFICATION

(11) **EP 1 981 978 B1**
(45) Date of publication and mention of the grant of the patent: **19.01.2011**
(21) Application number: 07736519.5
(22) Date of filing: 07.02.2007
(51) Int. Cl.: C12N 15/62

(54) **AFFINITY POLYPEPTIDE FOR PURIFICATION OF RECOMBINANT PROTEINS**
AFFINITÄTSPOLYPEPTID ZUR REINIGUNG VON REKOMBINANTEN PROTEINEN
POLYPEPTIDE D'AFFINITÉ POUR LA PURIFICATION DE PROTÉINES RECOMBINANTES

(30) Priority: 08.02.2006 IN MU01862006
(43) Date of publication of application: 22.10.2008
(73) Proprietor: USV Limited, Govandi, Mumbai 400 088 MAH (IN)
(72) Inventor: RAO, Laxmi, S., Maharastra (IN); MISHRA, Shrikant, Maharastra (IN); BORBHUIYA, Monsur, Ahmed, Maharastra (IN); MHATRE, Deepa, Maharastra (IN); THAKUR, Priti, Maharastra (IN)
(74) Representative: Schön, Christoph
(86) International application number: PCT/IN2007/000051
(87) International publication number: WO 2007/096899

(56) References cited:
- IVENS A C ET AL: "The genome of the kinetoplastid parasite, Leishmania major" SCIENCE, AMERICAN ASSOCIATION FOR THE ADVANCEMENT OF SCIENCE,, US, vol. 309, no. 5733, July 2005 (2005-07), pages 436-442,416, XP002400473 ISSN: 0036-8075 -& DATABASE UniProt [Online] 19 July 2005 (2005-07-19), "Putative uncharacterized protein." XP002463314 retrieved from EBI accession no. UNIPROT:Q4Q3Y7 Database accession no. Q4Q3Y7
- ARNAU ET AL: "Current strategies for the use of affinity tags and tag removal for the purification of recombinant proteins" PROTEIN EXPRESSION AND PURIFICATION, ACADEMIC PRESS, SAN DIEGO, CA, US, [Online] vol. 48, no. 1, July 2006 (2006-07), pages 1-13, XP005469149 ISSN: 1046-5928 Retrieved from the Internet: URL:available online Dec 2005> [retrieved on 2007-12-27]

## Description

### FIELD OF INVENTION

The present disclosure relates to the production of fusion recombinant proteins comprising biologically active polypeptide or protein of interest and affinity polypeptide, where the affinity polypeptide is useful for the purification of recombinant proteins using affinity chromatography on metal chelate resins with immobilized metal ions.

### BACKGROUND OF THE INVENTION

Genes for the desired protein may be isolated from organisms, which comprises of the gene found in nature or alternately they may be chemically synthesized. The isolated or chemically synthesized gene may be inserted in vectors and expressed in host systems, which produce the desired protein at high levels. Suitable purification procedures are indispensable for the establishment of an efficient process to obtain recombinant or synthetic proteins. It can be useful to know beforehand some physical properties such as hydrophobicity and ionic charge of the protein to facilitate the development of a suitable purification protocol from the recombinant source. On the other hand, there are now several ways of preparing fusion proteins, which can be purified by affinity techniques without any knowledge of the properties of the target protein. Using affinity handles as fusion partners, efficient purification schemes may be used which allows rapid recovery of expressed foreign gene products from crude extracts. By linking the coding gene sequence of the foreign protein to the coding gene sequence of a protein fragment having a high affinity for a ligand, it is possible to purify the foreign protein in the form of fusion proteins in one step using the affinity peptide. Hybrid products require a cleavage process for the liberation of the protein of interest from the precursor molecule for preparation of pharmaceutical grade recombinants. The cleavage could be either chemical or enzymatic. The use of 2-6 histidine (His) residues as an affinity tag has been described in patent US 5,310,663. Other bacterial gene products have been used which include staphylococcal protein A, the maltose-binding protein encoded by the *malE* gene in *E. coli* and glutathione-S-transferase of *Schistosoma japonicum* as disclosed in US Patent 5,935,824.

The starting material usually contains a large number of different proteins and the desired protein may be a major component especially if produced as the result of recombinant expression. The first stage should aim mainly to reduce the volume and get rid of as much non-protein material as possible. Purification procedures are commonly divided into 3 stages: a) the primary stage, which deal with crude mixtures of proteins and other molecules present in the raw material; b) the secondary stage, which generates a product near to homogeneity; and c) the polishing stage, which remove minor contaminants.

Genes from any source can be cloned and expressed in heterologous cells that can be cultured on a large scale in fermentor. Thus, any protein can, in principle, be produced in large quantities. The desired proteins must then be isolated and recovered from the total amount of protein produced by the host cells. This process can be very expensive and can cause denaturation of the protein product. Thus, there was a need to improve purification of these proteins from complex mixtures in highly efficient and predictable manner: Among the methods used to purify proteins, the most common are ion-exchange, hydrophobic and gel filtration chromatography. Since all these methods lack specificity, they are not suitable to achieve pure protein in high yields. In the case of recombinant DNA-derived proteins, it is difficult to predict selection of the purification methods for a given protein and a purification method has to be developed for each new protein since small changes in amino acid composition may change the protein solubility, shape, protein-protein interaction, hydrophobicity, and aggregation properties and hence affect purification protocols.

The desired proteins may be isolated from complex mixtures by methods based on differences in parameters such as size, solubility and charge. However none of these methods are capable of purifying proteins beyond a moderate level. The most common problem is low yield of purified protein vis-à-vis the expected fraction. This can be caused by insufficient protein loaded on the column, protein not binding to the column or protein not eluting from the column or nonspecific binding of other proteins to the column. Affinity chromatography based on the ability of proteins to bind non-covalently but specifically with an immobilized ligand is often preferred as it can purify proteins from complex mixtures without significant losses. The disadvantage is that it requires the availability of the corresponding ligand for the desired protein, which is not always possible since such specific ligands do not exist for all proteins.

To overcome this problem, antibodies to a linker peptide may be used as an immunoaffinity ligand. The fusion peptide is passed through a column containing immobilized antibodies which bind to the antigenic linker peptide and thus the fusion peptide is isolated (disclosed in US Patent No 4,782,137). The disadvantage of this method is that the desired polypeptide may get denatured by either the buffer conditions which are necessary to allow immunogenic complexing or the buffer conditions which must be present to terminate such complexes, leading to low yields of biological active protein.

Due to the difficulties often experienced in purifying recombinant proteins, a variety of vector systems (Sassenfeld, 1990) have been developed in which the expressed protein is a fusion protein containing an N-terminal polypeptide that simplifies purification. Such "tags" can be subsequently removed using a specific protease or by chemical cleavage. Tags used include proteins and polypeptides for which there is a specific antibody, binding proteins that will interact with columns containing a specific ligand, polyhistidine tags with affinity to immobilized metal columns and sequences that result in biotinylation by the host and enable purification on an avidin column.

Protein and peptide affinity tags have become highly popular tools for purifying recombinant proteins. They can provide hundred or even thousand-fold purification from crude extracts without prior steps to remove nucleic acid or other cellular material. J. Porath introduced immobilized metal ion affinity chromatography (IMAC) for fractionating proteins (Porath et al, 1975). IMAC consists of derivatizing a resin with iminodiacetic acid (IDA) and chelating metal ions to the IDA-derivatized resin. Proteins bind to the metal ions through amino acid residues capable of donating electrons and are immobilized on the column. Amino acids such as histidine, cysteine, methionine, arginine, glutamic acid, aspartic acid, lysine, tryptophan and tyrosine are responsible in part for the actual binding of free metal ions to such proteins. Most preferred immobilized metal ion chelating peptides are those containing histidine. The actual mechanisms which give rise to the binding of proteins to free metal ions are poorly understood and are dependent on a number of factors such as conformation of the protein, number of available coordination sites on the immobilized metal ion, accessibility of the protein side chains to the metal ion, number of available amino acids for coordination with the immobilized metal ion. Once binding has occurred, the protein can be released by protonation of its associated metal ion-binding ligand. Dissociation is achieved by lowering the pH of the surrounding buffer medium or using competitive counter ligands such as imidazole. Histidines containing di-or tri-peptides in proteins have been used to show that IMAC is a specific and selective purification procedure (US Patent No 4,569,794 and US Patent No 5,310,663).

Production of recombinant proteins with an affinity tag can serve three purposes. First, the affinity interaction can be used to immobilize the expressed protein on a solid support such as a biosensor or affinity column without any prior purification of the protein. Second, the immobilized fusion protein can be eluted and used directly for structural or functional studies or be used as an immunogen to generate antibodies. Third, the affinity-purified fusion protein can be processed by site-specific cleavage to release the desired product. When a fusion system is designed to facilitate protein purification, the structure of the affinity handle as well as the ligand must be designed to give an appropriate binding strength to allow elution of the fusion protein without denaturing the recombinant protein. Different gene fusion systems used for protein purification are shown in Table 1.

**Table 1: Gene Fusion Systems used to facilitate Protein Purification**

| **Gene Product** | **Origin** | **Ligand** |
|---|---|---|
| β-Galactosidase | *Escherichia coli* | p-aminophenyl-β-D-thiogalactosidase (TPEG), p-aminophenyl--β-D-thiogalactosidase (APTG) |
| Protein A | *Staphylococcus aureus* | IgG |
| Chloramphenicol Acetyl transferase (CAT) | *Escherichia coli* | Chloramphenicol |
| Poly (Arg) | Synthetic | Ion-exchange |
| Streptavidin | *Streptomyces* | Biotin |
| Z (IgG-binding fragment based on staphylococcal protein A) | Synthetic | IgG |
| PhoS (Phosphate-binding protein) | *Escherichia coli* | Hydroxylapatite |
| Protein G | *Streptococci* | Albumin |
| Maltose-binding protein (MBP) | *Escherichia coli* | Starch |
| Glutathione S-transferase (GST) | *Escherichia coli* | Glutathione |
| Flag peptide | Synthetic | Specific IgG |
| Poly (His) | Synthetic | Cu²⁺, Zn²⁺ |

There are two ways to obtain specific cleavage of desired proteins from the fusion partner: chemical (e.g. cyanogen bromide, formic acid, hydroxylamine etc) and enzymatic (e.g. Factor Xa, Thrombin, Enterokinase, Trypsin, human renin, carboxypeptidase A, dipeptidyl peptidase etc). The chemical methods cleave after a single amino acid or between residues in a dipeptide sequence.

The low specificity limits their use because the recombinant product frequently contains the corresponding residue or peptide. Sequences recognized by some enzymes can be more specific. Enzymatic cleavages are more affected by steric factors as compared to chemical methods and hence the cleavage site must be carefully engineered to be structurally accessible to the enzyme.

Amau et al. describe in "Current strategies for the use of affinity tags and tay removal for the purification of recombinant proteins", Protein Expression and Purification, Academic Press, San Diego, CA, US, Viol. 48 No. 1, July 2006, pages 1-13 various tags and purification systems in a review about protein purification based on tagged proteins.

### SUMMARY OF THE INVENTION

The present disclosure provides an affinity polypeptide for the purification of a recombinant biologically active protein or polypeptide. The disclosure relates to a recombinant expression vector for the production of fusion recombinant protein in host cells. Further, the present disclosure provide a process of producing fusion recombinant protein or polypeptide wherein the fusion recombinant protein comprises of at least two components, a biologically active polypeptide or protein (recombinant protein) and an affinity polypeptide. Further, the present disclosure provides an improved method of purification of recombinant protein from the host cells. Further, the disclosure provides a method of purification of the recombinant biologically active polypeptide/ protein by immobilized metal ion chelate chromatography.

According to the present invention these are provided:
1. An affinity polypeptide of general formula R.sup. 1-T-R.sup.2, wherein R.sup.1 is a peptide comprising 1-30 amino acid(s); T having general formula (Xₙ-His-Xₙ-Yₙ-Pro-Hisₙ)₂₋₆₀ wherein, X is selected from a group consisting of Gly, Ala, Ser, Thr, Asp, Glu, His, Val, and Leu; Y is selected from the group consisting of Gly, Ser, Glu, Asp, Lys, Val, Arg, Leu and Thr; n ranges from I to 4; and R.sup.2 is Z, or Z-Asp-Asp-Asp-Asp-Lys- or Z-Ile-Glu-Gly-Arg-, where Z is a peptide ranging from 1 to 30 amino acid(s).
2. The polypeptide as described in item 1, having the amino acid sequence as shown in SEQ ID NO: 1, SEQ ID NO: 2, SEQ ID NO: 3 or SEQ ID NO: 4.
3. A polypeptide represented as T having amino acid sequence as shown in SEQ ID NO: 5, SEQ ID NO: 6, SEQ ID NO: 7 or SEQ ID NO: 8, wherein T has a general formula (Xₙ-His-Xₙ-Yₙ-Pro-Hisₙ)₂₋₆₀ wherein, X is selected from a group consisting of Gly, Ala, Ser, Thr, Asp, Glu, His, Val, and Leu; Y is selected from the group consisting of Gly, Ser, Glu, Asp, Lys, Val, Arg, Leu and Thr; n ranges from 1 to 4.
4. A polynucleotide sequence encoding the polypeptide as described in item I or 2, wherein, if encoding the polypeptide as claimed in item 2, the nucleotide sequence is as shown in SEQ ID NO: 9, SEQ ID NO: 10, SEQ ID NO: 11 or SEQ ID NO: 12.
5. A polynucleotide sequence encoding polypeptide represented as T as described in item 3, wherein the nucleotide sequence is as shown in SEQ ID NO: 13, SEQ ID NO: 14, SEQ ID NO: 15 or SEQ ID NO: 16.
6. A recombinant expression vector comprising a promoter, polynucleotide sequence as described in item 4, a protease recognition site or a chemical cleavage site and a heterologous DNA of interest.
7. The recombinant expression vector as described in item 6, wherein said promoter is selected from a group consisting of AraB, trp, tac, lac, osmB, CMV, EF-1α, SV 40 and T7.
8. The recombinant expression vector as described in item 6, wherein said protease recognition site is recognized by protease selected from a group consisting of Enterokinase, Carboxy peptidase, Factor Xa, Trypsin, V8, and Chymotrypsin, preferably Enterokinase.
9. The recombinant expression vector as described in item 6, wherein said chemical cleavage site is recognized by a reagent selected from a group consisting of cyanogen halide, hydroxyl amine, formic acid, acetic acid, hydrochloric acid and trifluoroacetic acid.
10. The recombinant expression vector as described in item 6, wherein said heterologous DNA of interest is selected from a group consisting of DNA coding for β-galactosidase, Granulocyte Colony Stimulating Factor, human Interleukin, Interleukin-2, Platelet-derived growth factor, Granulocyte Macrophage Colony Stimulating Factor, Insulins, bovine Enterokinase, Vascular Endothelial Growth Factor, Nerve Growth Factor, Interferons and Tissue Plasminogen Activators.
11. A host cell comprising the recombinant expression vector as described in item 6 preferably a host cell having the Accession No. MTCC 5312.
12. The host cell as described in item 11, wherein said host cell is selected from a group consisting of E. coli, Pseudomonas, Yeast, Saccharomyces, Pichia, Hanseneula, CHO, BHK and COS, preferably the host cell being E. coli, more preferably said E. coli being selected from a group consisting of JM109, DH5α, Top10, BL21, HB101, XL1-Blue and LMG19.
13. A process for the production and purification of a recombinant protein, said process comprising;
   obtaining and growing recombinant cells having the recombinant expression vector as described in item 6 in a suitable medium for the production of the recombinant protein; recovering said recombinant protein by a single step method.
14. The process as described in item 13, wherein said recombinant protein is selected from a group consisting of β-galactosidase, Granulocyte Colony Stimulating Factor, human Interleukin, Interleukin-2, Platelet-derived growth factor, Granulocyte Macrophage Colony Stimulating Factor, Insulins, bovine Enterokinase, Vascular Endothelial Growth Factor, Nerve Growth Factor, Interferons and Tissue Plasminogen Activators.
15. The process as described in item 13, wherein said recovery of recombinant protein is done by purification using immobilized metal ion chelating chromatography.
16. The process as described in item 15, wherein said immobilized metal ion chelating chromatography employs metal ion selected from a group consisting of Ni, Cu, Zn and Co.

### BRIEF DESCRIPTION OF FIGURES

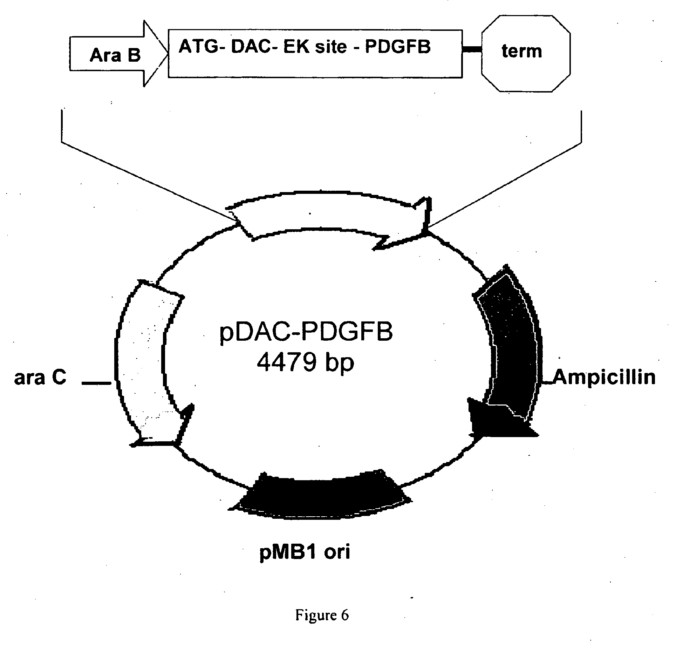

**Figure 1****:** Map of recombinant vector pDAC-lacZ
**Figure 2****:** Stick and ball model of the affinity handle, DAC, SEQ ID NO: 5 based on 3D PSSM analysis
**Figure 3****:** SDS-PAGE analysis showing binding ability and expression analysis of □-galactosidase protein
   Lane 1: HMW marker
   Lane 2: Total protein
   Lane 3: Supernatant
   Lane 4: Ni-IDA FT (-imidazole)
   Lane 5: Ni-IDA wash (-imidazole)
   Lane 6: Ni-IDA elution (-imidazole)
   Lane 7: Ni-IDA FT (+imidazole)
   Lane 8: Ni-IDA wash (+imidazole)
   Lane 9: Ni-IDA elution (+imidazole)
   Lane 10: Cu-IDA FT (-imidazole)
   Lane 11: Cu-IDA wash (-imidazole)
   Lane 12: Cu-IDA elution (-imidazole)
   Lane 13: Cu-IDA FT (+imidazole)
   Lane 14: Cu-IDA wash (+imidazole)
   Lane 15: Cu-IDA elution (+imidazole)

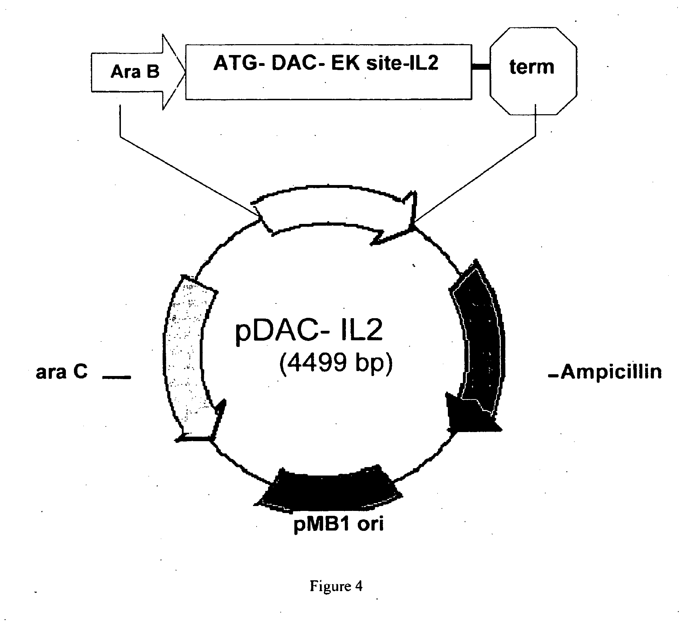

**Figure 4****:** Map of recombinant vector pDAC-IL2

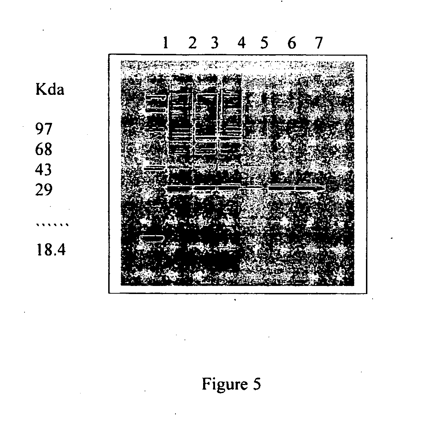

**Figure 5****:** SDS-PAGE showing the binding ability of expressed Interleukin
   Lane 1: LMW Marker
   Lane 2: Total lysate protein
   Lane 3: Ni-IDA - flow-through
   Lane 4: Ni-IDA - wash
   Lane 5: Ni-IDa - 40mM Imidazole elution
   Lane 6: Ni-IDa - 80mM Imidazole elution
   Lane 7: Ni-IDa - 250mM Imidazole elution
**Figure 6****:** Map of pDAC-PDGFB

### DETAILED DESCRIPTION OF THE INVENTION

The present disclosure provides an affinity polypeptide for the purification of a recombinant biologically active protein or polypeptide. The disclosure relates to a recombinant expression vector for the production of fusion recombinant protein in host cells. Further, the present disclosure provide a process of producing fusion recombinant protein or polypeptide wherein the fusion recombinant protein comprises of at least two components, a biologically active polypeptide or protein (recombinant protein) and an affinity polypeptide. The biologically active polypeptide may be linked directly or indirectly to the affinity polypeptide by covalent binding. Further, the present disclosure provides an improved method of purification of recombinant protein from the host cells. Further, the disclosure provides a method of purification of the recombinant biologically active polypeptide/ protein by immobilized metal ion chelating chromatography

### Definitions

The term "host cell" used herein means a cell which contains a vector and supports the replication and/or expression of the vector. Host cells may be prokaryotic cells such as *E. coli,* or eukaryotic cells such as yeast, insect, amphibian, or mammalian cells.

The term "expression vector" means a plasmid, yeast, or animal virus genome, used to introduce foreign genetic material into a host cell in order to replicate and amplify the foreign DNA sequences as a recombinant molecule. Once the expression vector is inside the host cell, the protein that is encoded by the gene is produced by the cellular transcription and translation machinery.

The terms "peptide", "polypeptide", and "protein" are used interchangeably herein after to refer to a polymer of amino acid residues. Peptide is organic compound composed of amino acids linked together chemically by peptide bonds. The peptide bond is a single covalent bond between the α-carboxyl (oxygen-bearing carbon) of one amino acid and the amino nitrogen of a second amino acid. Small peptides with fewer than about ten constituent amino acids are called oligopeptides, and peptides with more than ten amino acids are termed polypeptides. Protein is molecule made up of amino acids that are needed for the body to function properly.

The term "heterologous gene" or "heterologous DNA" used herein means a nucleic acid or a DNA sequence that originates from a foreign species, or, if from the same species, is considerably modified from its native form in composition and/or genomic locus by human intervention.

The term "promoter" used herein refers to a region of DNA upstream from transcription start site that is involved in recognition and binding of RNA polymerase and other proteins to initiate and/or to regulate transcription of the gene or heterologous DNA.

"Affinity tag peptide" or "affinity polypeptide" and "affinity handle" are used interchangeably herein to refer to a polypeptide sequence used for the purification of recombinant protein.

"Fusion recombinant protein" refers to a protein that comprises at least two components, a biologically active polypeptide or protein of interest (recombinant protein) and an affinity polypeptide.

"Recombinant protein" or "Recombinant polypeptide" refers to the protein or polypeptide of interest.

The term "immobilized metal ion chelating peptide" as used herein means an amino acid sequence that chelates immobilized divalent metal ions of metals selected from the group consisting of nickel, copper, zinc and cobalt.

An embodiment of the present disclosure provides an affinity polypeptide of general formula R.sup.1-T- R.sup.2, where R.sup.1 is a peptide comprising 1-30 amino acid(s); T having general formula (Xₙ-His-Xₙ-Yₙ-Pro.Hisₙ) _{2 -60} where, X is selected from a group consisting of Gly, Ala, Ser, Thr, Asp, Glu, His, Val, and Leu; Y is selected from the group consisting of Gly, Ser, Glu, Asp, Lys, Val, Arg, Leu and Thr; n ranges from 1 to 4; and R.sup.2 is Z, or Z-Asp-Asp-Asp-Asp-Lys- or Z-Ile- Glu-Gly-Arg-, where Z is a peptide ranging from 1 to 30 amino acid(s).

Another embodiment of the present disclosure provides an affinity polypeptide having the amino acid sequence as shown in SEQ ID NO: 1.

Another embodiment of the present disclosure provides an affinity polypeptide having the amino acid sequence as shown in SEQ ID NO: 2.

Another embodiment of the present disclosure provides an affinity polypeptide having the amino acid sequence as shown in SEQ ID NO: 3.

Another embodiment of the present disclosure provides an affinity polypeptide having the amino acid sequence as shown in SEQ ID NO: 4.

Still another embodiment of the present disclosure provides an affinity polypeptide represented as T having amino acid sequence as shown in SEQ ID NO: 5.

Another embodiment of the present disclosure provides an affinity polypeptide represented as T having amino acid sequence as shown in SEQ ID NO: 6.

Another embodiment of the present disclosure provides an affinity polypeptide represented as T having amino acid sequence as shown in SEQ ID NO: 7.

Another embodiment of the present disclosure provides an affinity polypeptide represented as T having amino acid sequence as shown in SEQ ID NO: 8.

Yet another embodiment of the present disclosure provides an isolated polynucleotide sequence encoding said affinity polypeptide of general formula R.sup.1-T- R.sup.2, where R.sup.1 is a peptide comprising 1-30 amino acid(s); T having general formula (Xₙ-His-Xₙ-Yₙ-Pro.Hisₙ) ₂₋₆₀ where, X is selected from a group consisting of Gly, Ala, Ser, Thr, Asp, Glu, His, Val, and Leu; Y is selected from the group consisting of Gly, Ser, Glu, Asp, Lys, Val, Arg, Leu and Thr; n ranges from 1 to 4; and R.sup.2 is Z, or Z-Asp-Asp-Asp-Asp-Lys- or Z-Ile- Glu-Gly-Arg-, where Z is a peptide ranging from 1 to 30 amino acid(s).

Another embodiment of the present disclosure provides an isolated polynucleotide sequence encoding the affinity polypeptide having the nucleotide sequence as shown in SEQ ID NO: 9.

Another embodiment of the present disclosure provides an isolated polynucleotide sequence encoding the affinity polypeptide having the nucleotide sequence as shown in SEQ ID NO: 10.

Another embodiment of the present disclosure provides an isolated polynucleotide sequence encoding the affinity polypeptide having the nucleotide sequence as shown in SEQ ID NO: 11.

Another embodiment of the present disclosure provides a polynucleotide sequence encoding the affinity polypeptide having the nucleotide sequence as shown in SEQ ID NO: 12.

Still another embodiment of the present disclosure provides a polynucleotide sequence encoding an affinity polypeptide represented as T.

Another embodiment of the present disclosure provides a polynucleotide sequence encoding said affinity polypeptide represented as T having the nucleotide sequence as shown in SEQ ID NO: 13.

Another embodiment of the present disclosure provides a polynucleotide sequence encoding said affinity polypeptide represented as T having the nucleotide sequence as shown in SEQ ID NO: 14.

Another embodiment of the present disclosure provides a polynucleotide sequence encoding said affinity polypeptide represented as T having the nucleotide sequence as shown in SEQ ID NO: 15.

Another embodiment of the present disclosure provides a polynucleotide sequence encoding said affinity polypeptide represented as T having the nucleotide sequence as shown in SEQ ID NO: 16.

Yet another embodiment of the present disclosure provides a recombinant expression vector comprising a promoter, polynucleotide sequence encoding said affinity polypeptide, a protease recognition site or a chemical cleavage site and a heterologous DNA of interest.

Another embodiment of the present disclosure provides a recombinant expression vector comprising a promoter, polynucleotide sequence encoding said affinity polypeptide set forth in SEQ ID NO: 9 or SEQ ID NO: 10 or SEQ ID NO: 11 or SEQ ID NO: 12, a protease recognition site or a chemical cleavage site and a heterologous DNA of interest.

Still another embodiment of the present disclosure provides a recombinant expression vector where said promoter is *AraB* or trp or tac or lac or osmB or CMV or EF-1α or SV 40 or T7.

Another embodiment of the present disclosure provides a recombinant expression vector where said protease recognition site is recognized by protease such as Enterokinase, Carboxy peptidase, Factor Xa, Trypsin, V8, and Chymotrypsin.

Another embodiment of the present disclosure provides a recombinant expression vector where said protease is Enterokinase.

Another embodiment of the present disclosure provides a recombinant expression vector where said chemical cleavage site is recognized by a reagent such as cyanogen halide, hydroxyl amine, formic acid, acetic acid, hydrochloric acid and trifluoroacetic acid.

Another embodiment of the present disclosure provides a recombinant expression vector where said heterologous DNA of interest codes for proteins such as β-galactosidase, Granulocyte Colony Stimulating Factor, human Interleukin, Interleukin-2, Platelet-derived growth factor, Granulocyte Macrophage Colony Stimulating Factor, Insulins, bovine Enterokinase, Vascular Endothelial Growth Factor, Nerve Growth Factor, Interferons and Tissue Plasminogen Activators.

Another embodiment of the present disclosure provides a recombinant expression vector where said vector is pDAC-LacZ or pDAC-IL2 or pDAC-PDGF or pDAC-GCSF or pDAC-EK or pDAC-hGH or pDAC-IFN or pDAC-EPO.

Yet another embodiment of the present disclosure provides a host cell comprising the said recombinant expression vector.

Another embodiment of the present disclosure provides a host cell where said host cell is *E. coli* or *Pseudomanas* or Yeast or *Saccharomyces* or *Pichia* or, *Hanseneula* or CHO or BHK or COS.

Another embodiment of the present disclosure provides a host cell where said host cell is *E. coli.*

Another embodiment of the present disclosure provides a host cell where said *E. coli* is JM109 or DH5α or Top 10 or BL21 or HB101 or XL1-Blue or LMG19.

Another embodiment of the present disclosure provides a host cell having Accession No. MTCC 5312.

Another embodiment of the present disclosure provides a recombinant strain of E. coli Top10 comprising the recombinant expression vector containing pDAC-LacZ construct designated as *Escherichia coli* (K12) USVDAC Top 10. The accession number assigned to *Escherichia coli* (K12) USVDAC Top 10 is MTCC 5312.

Still another embodiment of the present disclosure provides a process for the production and purification of a recombinant protein by obtaining and growing recombinant cells having said recombinant expression vector in a suitable medium for the production of the recombinant protein and recovering said recombinant protein by a single step method.

Another embodiment of the present disclosure provides a process where said recombinant expression vector is pDAC-LacZ or pDAC-IL2 or pDAC-PDGF or pDAC-GCSF or pDAC-EK or pDAC-hGH or pDAC-IFN or pDAC-EPO.

Another embodiment of the present disclosure provides recombinant protein selected from proteins such as β-galactosidase, Granulocyte Colony Stimulating Factor, human Interleukin, Interleukin-2, Platelet-derived growth factor, Granulocyte Macrophage Colony Stimulating Factor, Insulins, bovine Enterokinase, Vascular Endothelial Growth Factor, Nerve Growth Factor, Interferons and Tissue Plasminogen Activators.

Still another embodiment of the present disclosure provides recovery of said recombinant protein by purification using immobilized metal ion chelating chromatography.

Another embodiment of the present disclosure provides said immobilized metal ion chelating chromatography by employing metal ion such as Ni, Cu, Zn and Co.

The present disclosure provides a method of production of a fusion recombinant protein comprising at least two components, a biologically active polypeptide or protein (recombinant protein) and an affinity polypeptide. Further, the present disclosure also provides a method of purification of recombinant protein from a mixture containing the recombinant protein and impurities. The method of purification of the recombinant protein consists of contacting the fusion recombinant protein with a resin containing immobilized metal ions for a sufficient amount of time, selectively eluting the recombinant protein from the resin by optionally cleaving to separate the desired protein from the affinity polypeptide.

When the fusion recombinant protein according to the present disclosure is contacted with an immobilized metal ion containing resin, the fusion recombinant protein will be immobilized which will allow it to be separated from impurities. The conditions needed for purification described in the present disclosure does not denature the fusion protein. Thus, in few steps, it is possible to purify the final recombinant protein product in high yield.

The purification is based on the ability of certain amino acids acting as electron donors on the surface of proteins (histidine, tryptophan, tyrosine or phenylalanine) to bind reversibly to transition-metal ions that have been immobilized by a chelating group covalently bound to a solid support. Of these amino acids, histidine is quantitatively the most important in mediating the binding of most proteins to immobilized metal ions. Copper and Nickel ions have the greatest affinity for histidine (Nagakawa & Porath, 1989). The chelating groups that are commonly used are either iminodiacetic acid (IDA) or nitrilotriacetic acid (NTA). Also, the presence of proline residue before His residue in the peptide handle gives rise to steric properties of the affinity handle.

The present disclosure describes fusions of heterologous protein of interest with a metal chelating peptide handle (affinity polypeptide) and separation of the heterologous protein from contaminants on columns containing immobilized metal ions. The fusion recombinant protein will chelate at the metal chelating peptide linker to the immobilized metal ions. The contaminants freely pass through the column and can be removed. By changing the conditions of the column such as ionic charge and pH, the fusion recombinant protein can be released and collected in pure form. The fusion recombinant protein can be chemically cleaved. Alternatively, a proteolytic cleavage site may be optionally inserted between the portion of the fusion recombinant protein, which contains the desired recombinant protein and the affinity polypeptide. These can be cleaved with a protease, which results in efficient purification of the heterologous protein of interest (recombinant protein). Choice of the affinity chromatographic technique helps in handling any large batch purification for industrial purpose with fewer steps in purification and reduces the loss of the final product of interest, thus making the whole process cost effective. Using the affinity polypeptide and method of the present disclosure, high yield of the heterologous protein of interest may be recovered in pure form. For example, 70% yield was obtained in a single step for β-galactosidase when other *E. coli* proteins were removed, while 74% yield was obtained from a bacterial lysate. The yield obtained for IL-2 in a single step while separating from other bacterial proteins was 78%.

The nucleotide sequence coding for the affinity polypeptide in pDAC-LacZ is linked to a protease cleavage site which is Enterokinase (EK) recognition site. Other protease cleavage sites such as Carboxy peptidase, Factor Xa, Trypsin, V8, and Chymotrypsin can also be used. Further, a chemical cleavage site can be inserted which is linked to the DNA sequence coding for the affinity polypeptide wherein the chemical cleavage site is recognized by a reagent selected from a group consisting of cyanogen halide, hydroxyl amine, formic acid, acetic acid, hydrochloric acid and trifluoroacetic acid.

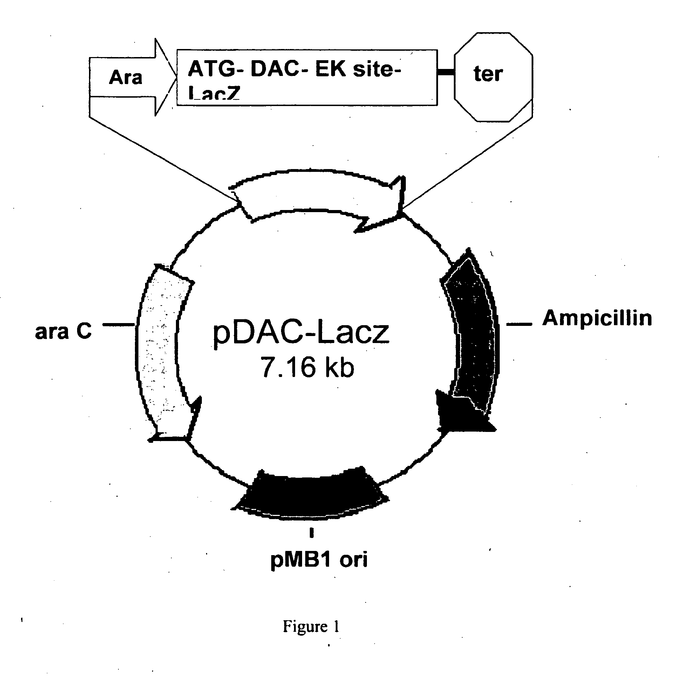

The nucleotide sequence coding for affinity polypeptide in the pDAC-LacZ vector is further linked to the *LacZ* gene under the control of the *AraB* promoter. The *LacZ* gene can also be driven by other promoter which is selected from the group consisting of trp, tac, lac, osmB, CMV, EF-1α, SV 40 and T7. The *LacZ* gene has the rrnB transcription terminator. The pDAC-LacZ vector also comprises of ampicillin ORF, pMB1 (pUC-derived) origin and AraC ORF. The detail procedure for the construction of the pDAC-LacZ is provided in Example 4. The schematic map of the pDAC-LacZ vector (7.16 kb) is depicted in Figure 1. The nucleotide sequence coding for an affinity polypeptide is located at nucleotide position 319 to 444 and is followed by a EK recognition site located at the nucleotide position 448 to 462. This is followed by *LacZ* gene which starts from nucleotide position 469 to 3428. The *LacZ* gene is terminated by rrnB terminator located at the nucleotide position 4526 to 3673.

The polynucleotide sequence of the insert containing the nucleotide sequence coding for affinity polypeptide, a EK recognition site, a portion of the DNA sequence coding for *LacZ* gene is shown in SEQ ID NO: 33. The present disclosure provides a recombinant pDAC-LacZ vector comprising the insert having polynucleotide sequence as shown in SEQ ID NO: 33. SEQ ID NO:33 provides the nucleotide sequence coding for an affinity polypeptide from nucleotide position 1 to 126 and is followed by a EK recognition site from nucleotide position 129 to 133. This is followed by DNA sequence coding for *LacZ* gene which starts from nucleotide position 151 onwards. The complete sequence coding for *LacZ* gene is not shown in the SEQ ID NO: 33 as it is well known in the art. The *LacZ* gene is followed by sequence for termination well known in the art.

the recombinant vector for producing the fusion recombinant protein comprises of the DNA sequence coding for the affinity polypeptide, a EK recognition site, a the DNA sequence coding for the protein or polypeptide of interest such as *LacZ* gene. The DNA coding for the affinity polypeptide may be selected from a group comprising of nucleotide sequence as shown in SEQ ID NO: 9, SEQ ID NO: 10, SEQ ID NO: 11 and SEQ ID NO: 12. The DNA sequence coding for EK recognition is well known in the art. The DNA coding for heterologous DNA of interest is selected from a group consisting of DNA coding for β-galactosidase, Granulocyte Colony Stimulating Factor, human Interleukin, Interleukin-2, Platelet-derived growth factor, Granulocyte Macrophage Colony Stimulating Factor, Insulins, bovine Enterokinase, Vascular Endothelial Growth Factor, Nerve Growth Factor, Interferons and Tissue Plasminogen Activators or any other biologically active polypeptide or protein or protein of interest.

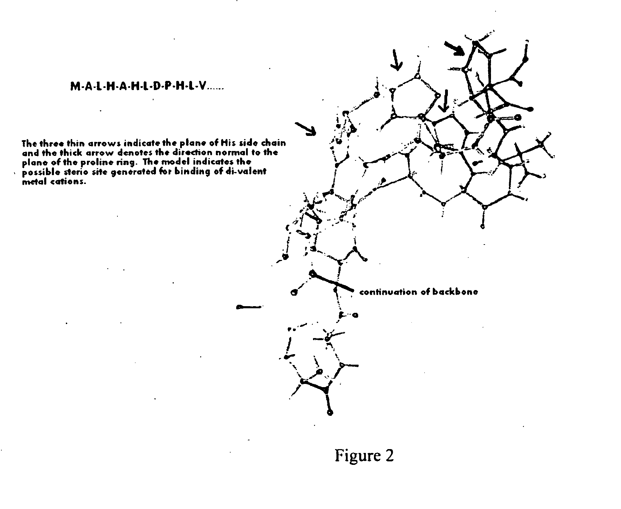

The Stick and Ball model generated for the first 11 amino acids of the affinity polypeptide, DAC, having SEQ ID NO: 5, based on the 3D PSSM analysis is provided in Figure 2. The three arrows in the figure indicate the plane of His side chain and the thick arrows denotes the direction normal to the plane of the Proline ring. The model indicates the possible stereo-site generated for the binding of di-valent metal cations. The Stick and Ball model, therefore confirms that the structure of the affinity polypeptide is best suited for use as an affinity handle for purification of fusion recombinant proteins compared to other affinity tags known in the art. The affinity polypeptide of the present disclosure is more efficient for the purification of the recombinant polypeptide or protein of interest. Further, the affinity polypeptide is easy to use for purification of recombinant proteins.

The present disclosure provides different methods of constructing the recombinant expression vectors for production of the fusion recombinant protein. The nucleotide sequence (SEQ ID NO: 9) coding for the affinity polypeptide (SEQ ID NO: 1) is first synthetically produced using the methods known in the art and inserted into the plasmid vectors to produce recombinant expression vector such as pDAC-LacZ. Other nucleotide sequences (SEQ ID NO: 10, SEQ ID NO: 11 and SEQ ID NO: 12) coding for affinity polypeptide (SEQ ID NO: 2, SEQ ID NO: 3 and SEQ ID NO: 4) can also be inserted into the plasmid vector using the methods known in the art to produce recombinant expression vector such as pDAC-LacZ.

The recombinant vector pDAC-LacZ was used to transform *strain* of *E. coli* cells such as JM109, DH5α, Top 10, BL21, HB101, XL1-Blue and LMG19 to produce recombinant *E. coli* strain. Transformation of *E. coli* strains with the desired recombinant expression vector such as pDAC-LacZ is given in Example 7. This vector can also be transformed into other host cells such as *Pseudomanas, Yeast, Saccharomyces, Pichia, Hanseneula,* CHO, BHK, COS and mammalian cells.

The recombinant pDAC-LacZ vector is responsible for the production of a fusion recombinant protein which comprises of at least two components, the affinity polypeptide and β galactosidase protein produced by the recombinant cells containing this vector.

The recombinant cells containing the pDAC-LacZ vector were grown in a suitable medium under conditions well known in the art to produce the fusion recombinant protein. Example 8 gives details of growing the recombinant cells. The fusion recombinant protein was purified by using immobilized metal ion chelating chromatography. Details of the purification are given in the Example 9. Pure β galactosidase protein was obtained from the fusion recombinant protein using protease or chemical cleavage method.

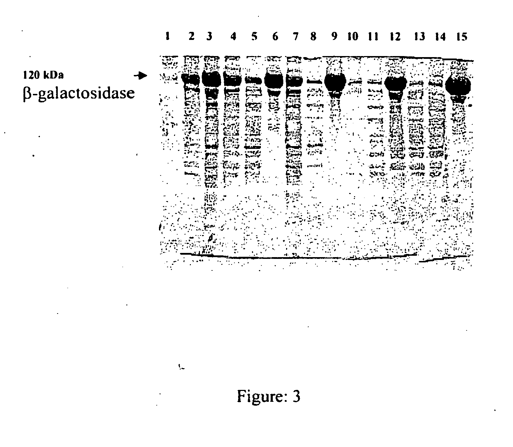

Plasmid DNA was from isolated the pDAC-LacZ vector and analyzed by restriction with various enzymes to confirm the vector construction. Details are given in Example 7. SDS-PAGE gel electrophoresis of β-galactosidase protein obtained from recombinant vector pDAC-LacZ was carried out. Details are given in Example 8. SDS-PAGE analysis (Figure 3) shows binding ability and expression analysis of β-galactosidase protein obtained from pDAC-LacZ. The presence of β-galactosidase proteins is observed in the flow-through and wash fractions. However, in case of Cu-IDA, less β-galactosidase protein was seen in flow-through and wash fractions showing that Cu-IDA and Ni- IDA has higher binding efficiency than Ni-IDA column.

Similarly, the recombinant expression vector such as pDAC-IL2 and pDAC-PDGFB were constructed as described above. The schematic map of the pDAC-IL2 vector (4499 bp) is depicted in Figure 4 and pDAC-PDGFB vector (4479 bp) is depicted in Figure 6. These vectors can also be constructed by methods well known in the art. The recombinant vector pDAC-IL2 has the nucleotide sequence coding for affinity polypeptide linked to chemical cleavage site at nucleotide position 322 to 444 which is further linked to DNA sequence of *IL-2* gene controlled by *AraB* promoter. The recombinant pDAC-PDGFB vector has the nucleotide sequence coding for the affinity polypeptide linked to EK site at nucleotide position 322 to 444 which is further linked to DNA sequence of *PDGFB* gene controlled by *AraB* promoter. These recombinant expression vectors were transformed into strains of *E. coli* cells such as JM109, DH5α, Top 10, BL21, HB101, XL1-Blue and LMG19 to produce recombinant *E. coli* strain. The recombinant cells containing these vectors were grown in the suitable medium under conditions as known in the art to produce fusion recombinant protein which was used to purify protein of interest (recombinant protein) by using immobilized metal ion chelating chromatography. Further, the desired protein was purified by chemical and or protease cleavage. The expression and binding analysis of pDAC-IL2, pDAC-PDGFB was carried out and details are explained in the Example 8-12. SDS-PAGE indicating Coomassie Stained gel of Ni-IDA purification showing the binding ability of expressed Interleukin in pDAC-IL2 is shown in Figure 5. Further, the expressed DAC-IL2 protein binds to Ni-IDA and is eluted with imidazole. Further, the elution of the bound protein is sensitive to imidazole concentration.

### Genomic DNA extraction from E. coli

Genomic DNA from *E. coli* (Top10, HB101, DH5 alpha and JM109 strains) was isolated. The details of the isolation of the genomic DNA from *E. coli* are given in Example 1.

### Construction of the plasmid vector containing the affinity polypeptide sequence

Amplification was carried out using specially designed primers (SEQ ID NO: 17 & 18 (25uM)), bacterial genomic DNA (Top10, HB101 and JM109 strains ) as template and DNA polymerase enzyme (1-2.5 units/µl) under following amplification conditions: 25-35 cycles of denaturation at 95°C for 1-5 min, annealing at 54-63°C for 1-5 min and extension at 72 °C for 1-10 min. The purified PCR product (designated as Fragment A) was used as a template for amplification with specifically designed primers having nucleotide sequence as shown in SEQ ID NO: 19 and SEQ ID NO: 20 ( See Example 1 for details ). The amplification methods are as described in Example 2.

The amplified fragment (designated as FRAGMENT B) and a prokaryotic expression vector such as pBAD/His, pET series, pRA, pcDNA3.1 Myc/His were digested with restriction enzymes *AgeI* and *SalI* in 1X buffer at 25-55°C overnight. The digested fragment (designated as FRAGMENT 1) was purified using PCR purification kit followed by ligation of the digested products with the restricted vector (designated as FRAGMENT 2) to create an intermediate vector (pTag-I) containing DNA sequence encoding the affinity polypeptide (see Example 2 for details).

The intermediate tag vector (pTag-I) was used as a template DNA for amplification with specific primers (SEQ ID NO: 21 and 22). The amplified fragment was digested with restriction enzymes *Age*I and *Sal*I to create a FRAGMENT 3 and a prokaryotic expression vector such as pRA was digested with the same enzymes i.e. *Age* I and *Sal* I to create FRAGMENT 4, in 1X buffer at 25-55°C overnight. Restricted FRAGMENT 3 and FRAGMENT 4 were extracted and purified using the PCR purification kit followed by ligation to produce a second intermediate vector (pRA-TagI) which was used as a template for another round of amplification (see Example 3 for details).

The above steps were repeated twice and the PCR product was purified and then digested over night with a restriction enzymes *Age* I and *Sal* I in respective 1X buffer at 25-55°C.

This was ligated to the digested expression vector such as pBAD/His, pET series, prokaryotic expression vector pRA, pcDNA3.1 Myc/His or other vectors known in the art to create the desired expression vector which has the DNA sequence for the affinity polypeptide linked to a protease cleavage site. A DNA sequence encoding for protein of interest (coding for proteins such as β-galactosidase, human growth hormone, human GCSF, human PDGFB, bovine enterokinase, human insulin, human interleukin, interferon and other clinically therapeutic protein or peptide) was linked to the protease cleavage site. The DNA sequences coding for biologically active polypeptide can be isolated from such vectors known in the art as pRA-LacZ, pRA-hGH, pRA-GCSF, pRA-PDGFB, pRA-EK, pRA-Insulin, and pRA-IL2.

To have more copies of the affinity polypeptide in tandem, the above steps can be repeated as many times (it is possible to have 1-10 copies of the affinity polypeptide in tandem).

The present disclosure provides construction of recombinant expression vector pDAC-LacZ vector which has the affinity peptide linked to EK site which is linked to DNA sequence for *LacZ* controlled by *AraB* promoter (See Example 4 for details).

Similarly, the expression vector pDAC-IL2 which comprises of the DNA sequences encoding the affinity polypeptide linked to EK site which is further linked to DNA sequences coding for IL2 protein. The DNA sequences in the vector pDAC-IL2 are under the control of *AraB* promoter. Other promoters known in the art may be employed for expression of the fusion recombinant protein. The details for producing the expression vector are provided in Example 5.

pDAC-PDGFB which has the affinity polypeptide linked to EK site which is further linked to DNA sequence of *PDGFB* gene controlled by *AraB* promoter were constructed (see Example 6 for details).

All of the above 3 constructs have the *Ara B* promoter with an Enterokinase protease cut site followed by the heterologous gene, *rrnB* transcription termination region, gene for ampicillin resistance, pMB1(pUC-derived) origin and the *Ara* C gene. These constructs (pDAC-LacZ, pDAC-IL2, pDAC-PDGFB) were transformed in competent cells of *E. coil* (DH5α, Top 10, LMG19, JM109 strains). Plasmid DNA was isolated and analyzed by restriction with various enzymes to confirm the vector construction. Details are given in Example 7. Expression analysis of pDAC-LacZ, pDAC-IL2, pDAC-PDGFB was carried out. The binding of the fusion recombinant proteins was carried out. Details are explained in the Example 8-12.

Enhanced genome annotation of the affinity polypeptide sequence was done using structural profiles in the programme 3D-PSSM (Kelley et al, 2000). The data was generated by the 3D-PSSM web server, Structural Bioinformatics group, Imperial College of Science, Technology and Medicine, UK. The predicted secondary structures that involve at least 3 amino acid residues binding to metal (e.g. Nickel) are selected from the E_score-predicted secondary structure of the sequence. E_scores are the probable structure predictors. From the Chou-Fasman analysis it is clear that two amino acid residues involved in metal binding belong to a seemingly alpha helical domain and one residue belongs to a turn.

Analysis of physicochemical properties of the affinity polypeptide was done using Protean module of Lasergene software. From the SDS-PAGE Simulation Analysis of protease cut sites of the affinity polypeptide using Protean module (Lasergene software, DNASTAR), it is seen that the affinity polypeptide is not cut by proteases like Enterokinase, Factor Xa, Trypsin and endopeptidase and hence the affinity polypeptide can be used in a genetic construct with either upstream or downstream cut sites of such proteases. Details are given in Example 13.

### EXAMPLES

It should be understood that the following examples described herein are for illustrative purposes only and that various modifications or changes will be suggested to a person skilled in the art

### Example I

### Isolation of genomic DNA and PCR amplification

Genomic DNA was isolated from *E. coli* strain JM109. 1.5 ml o/n bacterial culture (Top10, HB101, DH5-α and JM109) was transferred into a micro centrifuge tube and centrifuged at 6000 rpm, 5 min. Supernatant was decanted and the pellet was resuspended in TE buffer. SDS (Final 0.3%) and proteinase K was added, mixed well and incubated for 1 hr at 37°C. An equal volume of phenol/chloroform was added and mixed well by inverting the tube until the phases were completely mixed. DNA/phenol mixture was carefully transferred into a microcentrifuge tube and centrifuged for 2 min at 14 K rpm at room temperature. The upper aqueous phase was transferred to a new tube and an equal volume of phenol/chloroform was added, mixed well and centrifuged for 2 min at 14K rpm at RT. One tenth volume of sodium acetate was added to the upper aqueous phase which was transferred to a fresh microcentrifuge tube. DNA was precipitated by adding 0.6 volumes of isopropanol on ice for 5 to 8 minutes. DNA was pelleted by centrifugation at 14K rpm for 15 min at room temperature and washed with 70% ethanol. DNA was dissolved in 100-200 µl TE buffer kept at 37°C for 10-15 minutes.

Bacterial genomic DNA from JM109 strain of *E. coli* was used as the template for amplification using specially designed primers U-1 and L-1 (SEQ ID No: 17 and 18), and *Pfu* polymerase enzyme (1-2.5 units/µl, MBI) in 1X PCR buffer (MBI) containing 20mM Tris-HCl, pH 8.8, 10mM each of (NH₄)₂SO₄ and KCl, 0.1% Triton X-100, 0.1mg/ml BSA and 2mM MgSO₄ under following cycling conditions : 4 cycles of denaturation at 95°C for 1 min, annealing at 60°C for 2 min and extension at 72°C for 1min followed by 30 cycles of denaturation at 95°C for 1 min, annealing at 63°C for 2 min and extension at 72°C for 1 min. For analysis of the PCR products, 5-10 µl of sample was mixed with 1-10 µl of 1X loading dye and run on 1.0 % agarose gel.

| | |
|---|---|
| U-1 : 5' - GGTGTCGGCGATTTTTAACCGTGACTATC -3' | **SEQ ID NO: 17** |
| L-1:5' - CTGCCGCTACCGCCGACTAACG -3' | **SEQ ID NO: 18** |

Purified the 1099 bp PCR product (hereafter designated as Fragment A), using PCR purification kit. This purified fragment was used as the template for further amplifications.

### Example 2

### Preparation of pTag intermediate vector

Restriction enzyme site(s) was introduced during synthesis of the primers thus making possible the introduction of restriction enzyme sites at the 5' and 3' end of the amplified product. Fragment A was used as template for amplification with primers U-2 (has *Age* I site, SEQ ID No: 19) and L-2 (has *Sal* I site, SEQ ID No: 20). Amplification was carried out under the condition described in the Example 1.

| | |
|---|---|
| U-2: 5'-CTGGCGCTGACCGGTATCGCCCTGCTGGCGCTGATT -3' | **SEQ ID NO: 19** |
| L-2:5'-CGTAGTTGTCGACATGAGGGTCCAGGTGTGCGTGCTCCGTCA-3' | **SEQ ID NO: 20** |

Amplified product of 820 bp was purified using PCR purification kit and digested with restriction enzymes, *Age* I and *Sal* I, in buffer containing 50mM Tris-HCl, pH 7.5, 10mM MgCl₂, 100mM NaCl and 0.1mg/ml BSA at 37° C overnight. Fragment of 802 bp (hereafter designated as FRAGMENT 1) was purified using PCR purification kit. DNA sample was mixed with 3-5 volumes of the Buffer PB provided in the kit and applied to a column. This was centrifuged for 30-60 sec at 14K rpm and the flow through was discarded. The membrane was washed with the wash buffer provided and the DNA was eluted with nuclease free distilled water.

pRA DNA vector DNA was digested with *Age* I and *Sal* I in buffer containing 50mM Tris-HCl, pH 7.5, 10 mM MgCl2, 100 mM NaCl and 0.1 mg/ml BSA at 37°C overnight. The 3784 bp fragment (hereafter designated as FRAGMENT 2) and not the smaller 275 bp fragment, was purified as mentioned in Example 1. Ligation of FRAGMENT 1 and FRAGMENT 2 was carried out using 2 units of T4 DNA ligase in presence of buffer containing 40mM Tris HCl, pH 7.8, 10mM MgCl₂, 10mM DTT and 0.5mM ATP at 37° C for 2 hours and overnight at 4° C resulting in pTag-Intermediate vector (pRA-TAG-I), which was purified using PCR purification kit.

### Example 3

### Preparation of pRA-TAG-I vector

pTag-Intermediate vector was used as the template for amplification with primers U-3 (has *Nco* I site SEQ ID No: 21) and L-3 (has *Pvu* I site, SEQ ID No: 22) in a thermal cycler machine.

| | |
|---|---|
| U-3: 5'-GGTGGCCATGGCACTGCACGCACATCTGGACCCTCAT -3' | **SEQ ID NO: 21** |
| L-3: 5'- GTTCCCAACGATCAAGGCGAGTTACATGATC-3' | **SEQ ID NO: 22** |

This amplified product of 1160 bp was purified using PCR purification kit and digested with *Nco* I and *Pvu* I in buffer containing 33mM Tris-acetate, pH 7.9, 10mM Mg-acetate, 66mM Potassium acetate and 0.1mg/ml BSA at 37° C overnight. The 1080 bp fragment (hereafter designated as FRAGMENT 3) was purified using PCR purification kit.

pRA vector DNA was digested with *Nco* I and *Pvu* I in buffer containing 33mM Tris-acetate, pH 7.9, 10mM Mg-acetate, 66mM Potassium acetate and 0.1mg/ml BSA at 37°C overnight and 3012bp fragment (hereafter designated as FRAGMENT 4) was purified using PCR purification kit and not the smaller 1047 bp fragment. Ligation of FRAGMENT 3 with FRAGMENT 4 was set up using 2 units of T4 DNA ligase in presence of buffer containing 40mM Tris HCl, pH 7.8, 10mM MgCl₂, 10 mM DTT and 0.5mM ATP at 37° C for 2 hours and then overnight at 4° C to produce pRA-TAG-I vector.

### Example 4

### Preparation of pDAC-LacZ vector

pRA-Tag-I vector DNA was used as the template for amplification with primers U-4 (has *Age* I site, SEQ ID No: 23) and L-4 (has 6 more codons of the tag, SEQ ID No: 24) in a thermal cycler machine. Amplified product of 302 bp fragment (hereafter designated as FRAGMENT 5) was purified using PCR purification kit.

| | |
|---|---|
| U-4: 5'- GCTAACCAAACCGGTAACCCCGCTTATTAA- 3' | **SEQ ID NO: 23** |
| L-4: 5'-GGCGTGCTCCGTCACCAGATGAGGGTCCAGATGT- 3' | **SEQ ID NO: 24** |

pRA-Tag-I vector DNA was used as the template for amplification with primers U-5 (has 11 codons of the tag, SEQ ID No: 25) and L-5 (SEQ ID No: 26). Amplified product of 870 bp fragment (hereafter designated as FRAGMENT 6) was purified using PCR purification kit.

| | |
|---|---|
| U-5:5'-CTGGTGACGGAGCACGCCCACCTCGATCCGCACGTCGACGA-3' | **SEQ ID NO: 25** |
| L-5:5'-GGATAATACCGCGCCACATAGCAGAACTTTAAAAGTGCT-3' | **SEQ ID NO: 26** |

Purified FRAGMENT 5 and FRAGMENT 6 were used as the templates for amplification with primers U-6 having *Age* I site (SEQ ID No: 27) and L-6 having *Hind* III site (SEQ ID No: 28). Amplified product of 630 bp was purified and digested with *Age* I and *Hind* III in buffer containing 10mM Tris-HCl, pH 7.5, 10mM MgCL₂, 100mM NaCl and 0.1mg / ml BSA at 37° C overnight. The digested 382 bp fragment (hereafter named as FRAGMENT 7) was purified using PCR purification kit and not the smaller 248 bp fragment.

| | |
|---|---|
| U-6: 5'-CTAACCAAACCGGTAACCCCGCTTATTAAA- 3' | **SEQ ID NO: 27** |
| L-6: 5'-CCAGTCTTTCGACTGAGCCTTTCGTTTTATT- 3' | **SEQ ID NO: 28** |

pRA vector DNA was digested with *Age* I and *Hind* III in buffer containing 10mM Tris-HCl, pH 7.5, 10mM MgCL₂, 100mM NaCl and 0.1mg / ml BSA at 37° C overnight and 3710 bp fragment was purified using PCR purification kit (hereafter designated as FRAGMENT 8) and not the smaller 349 bp fragment. Ligation of FRAGMENT 7 with FRAGMENT 8 was carried out using 2 units of T4 DNA ligase in presence of buffer containing 40 mM Tris HCl, pH 7.8, 10 mM MgCl₂, 10mM DTT and 0.5mM ATP at 37°C for 2 hours and overnight at 4°C to produce pRA-T vector of size 4092 bp.

pRA-T vector DNA was digested with *Age* I and *Sal* I in buffer containing 50 mMTris-HCl, pH 7.5, 10 mM MgCl₂, 100 mM NaCl and 0.1 mg/ml BSA at 37°C overnight and 308 bp fragment (hereafter designated as FRAGMENT 9) was purified using PCR purification kit and not the larger 3784 bp fragment. pRA-LacZ vector DNA was digested with *Age* I and *Sal* I in buffer containing 50 mMTris-HCl, pH 7.5, 10 mM MgCl₂, 100 mM NaCl and 0.1mg/ml BSA at 37° C overnight and 6791 bp fragment was purified using PCR purification kit (hereafter designated as FRAGMENT 10) and not the smaller 275 bp fragment. FRAGMENT 9 was ligated with FRAGMENT 10 using 2 units of T4 DNA ligase in presence of buffer containing 4mM Tris HCl, pH 7.8, 10 mM MgCl₂, 10 mM DTT and 0.5 mM ATP at 37° C for 2 hours and overnight at 4°C to produce pT-LacZ vector (7099 bp). The purified ligated product was used as a template for amplification using specifically designed primers.

pT-LacZ vector DNA was used as the template, for amplification using primers U-7 (has. *Age* I site, SEQ ID No: 29) and L-7 (has *Nhe* I site, SEQ ID No: 30) in a thermal cycler machine.

| | |
|---|---|
| U-7:5'- GCTCTTCTCGCTAACCAAACCGGTAAC -3' | **SEQ ID NO: 29** |
| L-7:5'- CGTCGTCGCTAGCGTGCGGATCGAGGT -3' | **SEQ ID NO: 30** |

Amplified product of 339 bp was purified PCR purification kit and digested with *Age* I and *Nhe* I in buffer containing 33mM Tris-acetate, pH 7.9, 10mM Mg-acetate, 66mM K-acetate and 0.1mg/ml BSA at 37°C overnight. Fragment of 311 bp was purified using PCR purification kit (hereafter named as FRAGMENT 11).

pT-LacZ vector DNA was used as the template for amplification using primers U-8 (has *Nhe* I site, SEQ ID No: 31) and L-8 (has *Aat* II site, SEQ ID No: 32).

| | |
|---|---|
| U-8:5'-GGAGGAATTAACCGCTAGCCTGCACGCACATCTGGA-3' | **SEQ ID NO: 31** |
| L-8: 5'-GCAGCAACGAGACGTCACGGAAAAT-3' | **SEQ ID NO: 32** |

Amplified product of the 733 bp was purified using PCR purification kit and digested with *Nhe* I and *Aat* II in buffer containing 33 mM Tris-acetate, pH 7.9, 10 mM Mg-acetate, 66 mM K-acetate and 0.1mg/ml BSA at 37°C overnight and 704 bp fragment was purified (fragment hereafter designated as FRAGMENT 12).

FRAGMENT 11 with FRAGMENT 12 were ligated using 2 units of T4 DNA ligase in presence of buffer containing 40 mM Tris HCl, pH 7.8, 10 mM MgCl₂, 10 mM DTT and 0.5 mM ATP at 37°C for 2 hours and overnight at 4°C and 1015 bp ligated fragment was purified using PCR purification kit (hereafter designated as FRAGMENT 13).

pT-LacZ vector DNA was digested with *Age* I and *Aat* II in buffer containing 33mM Tris-acetate, pH 7.9, 10 mM Mg-acetate, 66 mM K-acetate and 0.1mg/ml BSA at 37°C overnight and 6146 bp fragment was purified using PCR purification kit (hereafter designated as FRAGMENT 14) and not the smaller 953 bp fragment. FRAGMENT 13 and FRAGMENT 14 were ligated using 2 units of T4 DNA ligase in presence of buffer containing 40 mM Tris HCl, 10 mM MgCl₂, 10 mM DTT and 0.5 mM ATP (pH 7.8) at 37° C for 2 hours and overnight at 4°C to produce pDAC-LacZ vector (7162 bp) which has the DNA sequence for affinity tag peptide (T) linked to EK site which is further linked to *LacZ* gene controlled by *AraB* promoter.

### Example 5

### Preparation of pDAC-IL2 vector

pDAC-LacZ vector DNA was digested with *Sal* I and *Hind* III in buffer containing 33 mM Tris-acetate, pH 7.9, 10 mM Mg-acetate, 66mM K-acetate and 0.1mg/ml BSA at 37°C overnight and the 4081 bp fragment (hereafter designated as FRAGMENT 15) was purified using PCR purification kit and not the smaller 3081 bp fragment. The recombinant vector pRA-IL2 was digested with *Sal* I and *Hind* III in buffer containing 33 mM Tris-acetate, pH 7.9, 10 mM Mg-acetate, 66 mM K-acetate and 0.1 mg/ml BSA at 37°C overnight and the smaller 419 bp fragment (hereafter designated as FRAGMENT 16) was purified using PCR purification kit and not the larger 3984 bp fragment. Ligation of FRAGMENT 15 with FRAGMENT 16 was carried out using 2 units of T4 DNA ligase in presence of buffer containing 40 mM Tris HCl, pH 7.8, 10 mM MgCl₂, 10 mM DTT and 0.5 mM ATP at 37°C for 2 hours and overnight at 4°C to produce pDAC-IL2 vector (4499 bp) which has the affinity polypeptide linked to EK site which is further linked to *IL2* gene controlled by *AraB* promoter.

### Example 6

### Preparation of pDAC-PDGF vector

pDAC-LacZ vector DNA was digested with *Sal* I and *Hind* III in buffer containing 33 mM Tris-acetate, pH 7.9, 10 mM Mg-acetate, 66 mM K-acetate and 0.1mg/ml BSA at 37°C overnight and the 4081 bp fragment (hereafter designated as FRAGMENT 15) was purified using PCR purification kit and not the smaller 3081 bp fragment. Recombinant vector pRAZ-1-PDGFB DNA was digested with *Sal* I and *Hind* III in buffer containing 33 mM Tris-acetate, pH 7.9, 10 mM Mg-acetate, 66 mM K-acetate and 0.1 mg/ml BSA at 37°C overnight and the smaller 402 bp (fragment hereafter designated as FRAGMENT 17) was purified using Qiaquick PCR purification kit and not the larger 4110 bp fragment. FRAGMENT 15 was ligated with FRAGMENT 17 using 2 units of T4 DNA ligase in presence of buffer containing 40 mM Tris HCl, pH 7.8, 10 mM MgCl₂, 10 mM DTT and 0.5 mM ATP at 37°C for 2 hours and overnight at 4°C to produce pDAC-PDGFB vector (4483 bp) which has the affinity polypeptide linked to EK site which is further linked to *PDGFB* gene controlled by *AraB promoter.*

Similarly, for producing a biologically active protein or peptide or protein of interest, recombinant vectors such as pDAC-GCSF, pDAC-EK, pDAC-hGH, pDAC-IFN and pDAC-EPO can be obtained by the employing the teachings of the present disclosure.

### Example 7

### Transformation of cells

The constructs such as pDAC-LacZ, pDAC-IL2 pDAC-PDGFB, and USVDAC-LacZ were transformed in competent cells of *E*. *coli* strains such as DH5δ, Top 10, LMG 19, JM109. Required amount of competent cells were thawed on ice. Ligation mix was transferred into the tube containing the competent cells, mixed gently without pipetting or vortexing and incubated on ice for 30 min. Cells were subjected to heat shock at 42°C / 2 mins and incubated on ice for 5 min. One ml of appropriate medium without antibiotic was added and cells were grown at 37°C/1hr with shaking. Cells were pelleted at 3000 rpm/5mins, resuspended in 100 µl appropriate medium without antibiotic and spread on an agar plate containing appropriate medium with antibiotic. Plates were incubated at 37°C in incubator for 12-18 hrs. Colonies obtained on the agar plate were inoculated in 3 ml liquid media containing antibiotic and allowed to grow at 37°C o/n with shaking. The recombinant strain of *E*. *coli* Top10 comprising the recombinant vector containing pDAC-LacZ construct was designated as *Escherichia coli* (K12) USVDAC Top 10. The accession number assigned to *Escherichia coli* (K 12) USDAC Top 10 is MTCC 5312.

### Plasmid isolation and analysis

Plasmid DNA was isolated by alkaline lysis method from 1.5ml o/n grown cultures by standard methods in prior art. DNA was subjected to restriction enzyme digestions to confirm the vector construction. The DNA of interest was cleaved with a variety of restriction endonucleases, either individually or in combination and the resulting products were separated by agarose gel electrophoresis. By determining the sizes of DNA fragments produced by the action of the endonucleases, the restriction map was deduced progressively from simple situations where enzymes cleave the DNA once or twice to more complex situations where cleavage occurs more frequently.

Positive clones were sequenced using the automated DNA sequencer (ABI Prism 310 Genetic Analyzer). The plasmid was purified either through columns or by PEG precipitation (*Kraft et al,* 1988). To the purified DNA (50 ng to 500 ng), 4-8 µl of the terminator ready reaction mix was added. This ready reaction mix is composed of premixed dNTPs, dye terminator, Taq DNA polymerase, MgCl₂ and buffer. On addition of 1 µl of primer (5 pmoles/µl), samples underwent cycle sequencing in a thermal cycler (25 cycles of 94°C for 10 sec, 50°C for 5 sec and 60°C for 4 mins). The resulting products were precipitated with 2.7 M sodium acetate (pH 4.6) and ethanol and washed twice with 70% ethanol. DNA pellet was dissolved in formamide. Samples were analyzed in the automated sequencer.

### Example 8

### Expression analysis of pDAC-LacZ, pDAC-IL2, pDAC-PDGF

The bacterial cells transformed with the recombinant expression vector (pDAC-LacZ, pDAC-IL2, pDAC-PDGF, pDAC-GCSF, pDAC-EK, pDAC-hGH, pDAC-IFN, pDAC-EPO) containing the affinity polypeptide and DNA sequence of interest (eg. β-galactosidase, IL2, PDGF) were grown in liquid media in presence of appropriate antibiotic. The cells were grown either till log (O.D ₆₀₀ₙₘ ∼ 0.5) or at a stationary phase (16 hrs growth) at 37°C in an incubator shaker in 1 liter fermentor. Then an appropriate amount (0-7%) of the inducer was added to the culture and incubated further for required time (0-72 hrs) at 37°C with shaking. The bacterial cells were harvested by pelleting at 4,000 rpm for 10 min at 4°C. The bacterial pellet was washed three times with ice-cold 1X PBS and resuspended in 200 µl of 1X PBS. Bacterial cell extracts were prepared by subjecting the cells to four cycles of rapid freezing in liquid nitrogen, followed by thawing at 37°C. Cells were vortexed vigorously for 5 min and centrifuged at 14,000 rpm for 10 min at 4°C. The supernatant was transferred to a fresh 1.5ml eppendorf tube. Total protein of the cell extract was estimated by Bradford Method. Colorimetric estimation of β-galactosidase protein was done using O-nitrophenyl β-D-Galacto-Pyranoside (ONPG, Sigma) as the substrate at 405 nm O.D. (Table: 2).

**Table 2: Expression levels of the reporter gene by enzymatic assay**

| Sample | β-galactosidase / ug protein |
|---|---|
| pDAC-LacZ, Uninduced, 4hrs | 0.7 |
| pDAC-LacZ, Induced with 0.2% L-Arabinose, 4hrs | 26.5 |

Cell pellets were sonicated in lysis buffer and centrifuged to get clear cell lysate. After protein content was estimated by Bradford method, the required amount of sample was mixed with the sample buffer and heated at 90°C for 5 min. The samples were pulse spun and loaded on a SDS-PAGE gel (10- 20%) immediately. After the electrophoretic run, the gel was stained with coomassie blue.

### Example 9

### Binding Studies of β-galactosidase protein

Cell pellets were resuspended in 25ml lysis buffer (50 mM Tris, pH 8.0, 150 mM NaCl) and sonicated for 10 min (30 sec on/off pulse mode). Cell lysates were centrifuged for 15min at 15,000 rpm and the clear supernatant was used for affinity chromatography. Twenty-five mg of clear supernatant was used for studying the binding of LacZ protein with the affinity polypeptide to metal affinity beads. Chelating Sepharose beads were packed in a 1ml PD-10 column. The beads were allowed to settle and washed with 10 ml distilled water, followed by 10 ml of 100mM NiSO₄ or CuSO₄, agin with 10 ml of distilled water and finally with 10ml of equilibration buffer (50mM Tris, pH 8.0, 150mM NaCl) with and without 20mM imidazole.

Sample (with and without 20mM imidazole) was loaded onto the column at 1 ml / min flow rate and washed with 5 ml of respective buffers (with and without 20mM imidazole) to remove unbound proteins. Bound protein in all experiments was eluted with 2 ml of respective buffer containing 250 mM Imidazole. Protein estimations of all relevant fractions were done using Bradford's protein assay kit from Pierce. Samples were analyzed on 12% SDS-PAGE gel followed by Coomassie staining (Table: 3).

**Table: 3 Protein estimations for pDAC-LacZ sample**

| Exptal. conditions | Conc. in mg/ml | Total vol in ml | Total protein in mg |
|---|---|---|---|
| Total Protein loaded onto the column | 8.07 | 3.09 | 25.0 |
| Ni-IDA elution ( -imidazole in eq. buffer) | 1.54 | 2 | 3.08 |
| Ni-IDA elution (+ imidazole in eq. buffer) | 1.66 | 2 | 3.32 |
| Cu-IDA elution (- imidazole in eq. buffer) | 2.49 | 2 | 4.98 |
| Cu-IDA elution (+ imidazole in eq. buffer) | 1.94 | 2 | 3.88 |

Binding efficiency of expressed β-galactosidase protein to Ni-IDA was found to be better when bound in presence of equilibrating buffer which does not contain Imidazole. SDS-PAGE analysis (Fig: 3) showed the presence of β-galactosidase proteins in flow-through and wash fractions. However, in case of Cu-IDA, less β-Galactosidase protein was seen in flow-through and wash fractions showing that Cu-IDA has higher binding efficiency than Ni-IDA although column. This shows that the affinity polypeptide of the present disclosure is highly efficacious for purification of recombinant protein or polypeptide or protein of interest. Further, the affinity polypeptide of the present disclosure can also be used for purification of other protein of interest, some of such examples are provided in the following examples.

### Example 10

### Analysis of pDAC-LacZ expression and binding

Cell pellets were resuspended in 25 ml lysis buffer (50mM Tris, pH 8.0, 150mM NaCl) and sonicated for 10 min (30 sec on/off pulse mode). Cell lysates were centrifuged for 15 min at 15,000 rpm and the clear supernatant was used for affinity chromatography. Clear supernatant of pDAC-LacZ (55 mg) was used for binding analysis. A 3 ml column of chelating Sepharose (Amersham Biosciences) was packed into an empty PD-10 column. The column was charged with 100 mM of CuSO₄ and equilibrated with 50mM Tris, pH 8.0, 150mM NaCl, 20mM imidazole. To the sample, imidazole was added to give 20mM final concentration. Sample was loaded onto the column at 1 ml/min flow rate. After complete loading, unbound proteins were washed off with the same buffer. Bound protein was eluted with above buffer containing 250 mM imidazole. Protein estimations of all relevant fractions were done using Bradford's protein assay kit (Pierce). The results are given in Table 4. Samples were analyzed on 12% SDS-PAGE electrophoresis. The gel was stained with Coomassie staining.

**Table: 4 Protein estimations for pDAC-LacZ clone sample**

| Sample | Conc. (mg/ml) | Total vol (ml) | Total protein (mg) |
|---|---|---|---|
| Total protein loaded onto the column | 5.5 | 10 | 55 |
| Cu-IDA elution | 1.12 | 6 | 6.72 |

### Example 11

### Expression analysis of pDAC-IL2

The bacterial cells transformed with the expression vector pDAC-IL2 were grown in liquid media containing ampicillin (50ug/ml). The cells were grown to stationary phase (16 hrs growth) at 37°C in an incubator shaker. An appropriate amount (0.2% final concentration) of L-Arabinose was added to the culture and incubated further for 7 hrs at 37°C with shaking. The bacterial cells were harvested by pelleting at 4000 rpm for 10 min at 4°C. Cell pellets were resuspended in 1.5ml lysis buffer (50 mM Tris, pH 8.0, 150mM NaCl). Samples were sonicated to disrupt cells and total lysate was spun at 15,000 rpm for 10 min. Protein was estimated from total lysate, supernatant and pellet. Ten µg of protein was analyzed on 15% SDS-PAGE and gel was stained with Coomassie stain.

### Example 12

### Binding studies of expressed DAC-IL2 protein

Cell pellets were solubilized in buffer containing 50mM Tris, pH 8.0, 150mM NaCl, 8M urea and 20mM imidazole for 3 hrs. 1 ml Ni-IDA beads were packed and charged with 5 column volumes of 0.1M Ni-SO₄. The column was equilibrated with equilibration buffer (50mM Tris, pH 8.0, 150mM NaCl, 8M urea, and 20mM imidazole) and ∼5.5 mg protein was loaded into the column. Flowthrough was collected. Column was washed with 2 column volumes of equilibration buffer and protein was eluted with elution buffer containing 250mM imidazole. Amount of protein in the different fractions was estimated and analyzed by SDS-PAGE electrophoresis (Table: 5).

**Table: 5 Protein estimations for pDAC-IL2 sample**

| Sample | Concentration (mg/ml) | Volume in ml | Total protein (mg) |
|---|---|---|---|
| Ni-IDA Starting material | 3.05 | 1.8 | 5.49 |
| Flowthrough/ Wash | 0.77 | 2 | 1.54 |
| Elution | 3.5 | 1 | 3.5 |

The expressed DAC-IL2 protein binds to Ni-IDA affinity matrix. Non-specific bound protein is removed mostly by a 40mM imidazole wash while the desired protein is eluted with buffer containing 80 and 250mM imidazole.

### Example 13

Enhanced genome annotation of the tag sequence was done using structural profiles in the programme 3D-PSSM (Kelley et al, 2000). The data was generated by the 3D-PSSM web server, Structural Bioinformatics group, Imperial College of Science, Technology and Medicine, UK. The predicted secondary structures that involve at least 3 amino acid residues binding to metal (eg Nickel) are selected from the E_score-predicted secondary structure of the sequence. E_scores are the probable structure predictors. From the Chou-Fasman analysis it is clear that two amino acid residues involved in metal binding belong to a seemingly alpha helical domain and one residue belongs to a turn. Physicochemical properties of the affinity tag peptide was analyzed using Protean software (Lasergene) and is given below -

Predicted Structural Class of the Whole Protein: R (Deleage & Roux Modification of Nishikawa & Ooi 1987)
Molecular Weight: 4589.06 m.w.
Length: 41 amino acids
Strongly basic (+) amino acids (K, R): 0
Strongly acidic (-) amino acids (D, E): 6
Hydrophobic amino acids (A, I, L, F, W, and V): 16
Polar amino acids (N, C, Q, S, T, Y):3
1 microgram =217.910 pmoles
Molar Extinction coefficient: 0 ± 5%
1 A(280): No CYWs
Isoelectric Point: 6.30
Charge at pH 7: -4.09
Results from Predictprotein (www.predictprotein.org) gave the following MAXHOM alignment.
Identities computed with respect to: (1) predict_h2630
Colored by: consensus / 70% and property

| | | |
|---|---|---|
| 1 predict_h2630 | 100.0% | ALHAHLDPHLVTEHAHLDPHASLHAHLDPHLVTEHAHLDPH |
| 2 nikc_ecoli | 87.5% | ALRDHLDPHLVTEHAH............................................................ |
| Consensus /100% | | AL+s HLDPHLVTEHAH............................................................ |
| Consensus /90% | | AL+s HLDPHLVTEHAH............................................................ |
| Consensus /80% | | AL+s HLDPHLVTEHAH............................................................ |
| Consensus /70% | | AL+sHLDPHLVTEHAH............................................................ |

The above results indicate that a portion of the affinity tag peptide (affinity handle) has 87.5% homology to *E*. *coli* nik c protein. A portion of the sequence information was used to build Fragment A.

### References:

Kelley, L.A., MacCallum, R.M. and Sternberg; M. J. E, (2000), J. Mol. Biol. 299 (2): 499-520.
Kraft et al, Biotechniques (1988) 6: 544-547
Porath, J., Carlsson, J., Olsson, I. and Belfrage, G., (1975), Nature, 258: 598-599.
Sassenfeld, H.M., (1990), Trends Biotechnol. 8: 88-93.

## Claims

1. An affinity polypeptide of general formula R.sup. 1-T-R.sup.2, wherein R.sup.l is a peptide comprising 1-30 amino acid(s) ; T having general formula (Xₙ-His-Xₙ-Yₙ-Pro-Hisₐ) ₂₋₆₀ wherein, X is selected from a group consisting of Gly, Ala, Ser, Thr, Asp, Glu, His, Val, and Leu; Y is selected from the group consisting of Gly, Ser, Glu, Asp, Lys, Val, Arg, Leu and Thr; n ranges from 1 to 4; and R.sup.2 is Z, or Z-Asp-Asp-Asp-Asp-Lys- or Z-Ile-Glu-Gly-Arg-, where Z is a peptide ranging from 1 to 30 amino acid(s) .

2. The polypeptide as claimed in claim 1, having the amino acid sequence as shown in SEQ ID NO: 1, SEQ ID NO: 2, SEQ ID NO: 3 or SEQ ID NO: 4.

3. A polypeptide represented as T having amino acid sequence as shown in SEQ ID NO: 5, SEQ ID NO: 6, SEQ ID NO: 7 or SEQ ID NO: 8, wherein T has a general formula (Xₙ-His-Xₙ-Yₙ-Pro-Hisₙ) ₂₋₆₀ wherein, X is selected from a group consisting of Gly, Ala, Ser, Thr, Asp, Glu, His, Val, and Leu; Y is selected from the group consisting of Gly, Ser, Glu, Asp, Lys, Val, Arg, Leu and Thr; n ranges from 1 to 4.

4. A polynucleotide sequence encoding the polypeptide as claimed in claim 1 or 2, wherein, if encoding the polypeptide as claimed in claim 2, the nucleotide sequence is as shown in SEQ ID NO: 9, SEQ ID NO: 10, SEQ ID NO: 11 or SEQ ID NO: 12.

5. A polynucleotide sequence encoding polypeptide represented as T as claimed in claim 3, wherein the nucleotide sequence is as shown in SEQ ID NO: 13, SEQ ID NO: 14, SEQ ID NO: 15 or SEQ ID NO: 16.

6. A recombinant expression vector comprising a promoter, polynucleotide sequence as claimed in claim 4, a protease recognition site or a chemical cleavage site and a heterologous DNA of interest.

7. The recombinant expression vector as claimed in claim 6, wherein said promoter is selected from a group consisting of AraB, trp, tac, lac, osmB, CMV, EF-1α, SV 40 and T7.

8. The recombinant expression vector as claimed in claim 6, wherein said protease recognition site is recognized by protease selected from a group consisting of Enterokinase, Carboxy peptidase, Factor Xa, Trypsin, V8, and Chymotrypsin, preferably Enterokinase.

9. The recombinant expression vector as claimed in claim 6, wherein said chemical cleavage site is recognized by a reagent selected from a group consisting of cyanogen halide, hydroxyl amine, formic acid, acetic acid, hydrochloric acid and trifluoroacetic acid.

10. The recombinant expression vector as claimed in claim 6, wherein said heterologous DNA of interest is selected from a group consisting of DNA coding for β-galactosidase, Granulocyte Colony Stimulating Factor, human Interleukin, Interleukin-2, Platelet-derived growth factor, Granulocyte Macrophage Colony Stimulating Factor, Insulins, bovine Enterokinase, Vascular Endothelial Growth Factor, Nerve Growth Factor, Interferons and Tissue Plasminogen Activators.

11. A host cell comprising the recombinant expression vector as claimed in claim 6, preferably a host cell having the Accession No. MTCC 5312.

12. The host cell as claimed in claim 11, wherein said host cell is selected from a group consisting of E.coli, Pseudomonas, Yeast, Saccharomyces, Pichia, Hanseneula, CHO, BHK and COS, preferably the host cell being E.coli, more preferably said E.coli being selected from a group consisting of JM109, DH5α, Top10, BL21, HB101, XL1-Blue and LMG19.

13. A process for the production and purification of a recombinant protein, said process comprising; obtaining and growing recombinant cells having the recombinant expression vector as claimed in claim 6 in a suitable medium for the production of the recombinant protein; recovering said recombinant protein by a single step method.

14. The process as claimed in claim 13, wherein said recombinant protein is selected from a group consisting of β-galactosidase, Granulocyte Colony Stimulating Factor, human Interleukin, Interleukin-2, Platelet-derived growth factor, Granulocyte Macrophage Colony Stimulating Factor, Insulins, bovine Enterokinase, Vascular Endothelial Growth Factor, Nerve Growth Factor, Interferons and Tissue Plasminogen Activators.

15. The process as claimed in claim 13, wherein said recovery of recombinant protein is done by purification using immobilized metal ion chelating chromatography.

16. The process as claimed in claim 15, wherein said immobilized metal ion chelating chromatography employs metal ion selected from a group consisting of Ni, Cu, Zn and Co.

## Patentansprüche

1. Affinitätspolypeptid der allgemeinen Formel R¹-T-R², worin R¹ ein Peptid mit 1 bis 30 Aminosäuren ist; T die allgemeine Formel (Xₙ-His-Xₙ-Yₙ-Pro-Hisₙ)₂₋₆₀ aufweist, worin X aus der Gruppe ausgewählt ist, die aus Gly, Ala, Ser, Thr, Asp, Glu, His, Val und Leu besteht; Y aus der Gruppe ausgewählt ist, die aus Gly, Ser, Glu, Asp, Lys, Val, Arg, Leu und Thr besteht; n in einem Bereich von 1 bis 4 liegt und R² für Z oder Z-Asp-Asp-Asp-Lys- oder Z-Ile-Glu-Gly-Arg- steht, worin Z ein Peptid im Bereich von 1 bis 30 Aminosäuren ist.

2. Polypeptid nach Anspruch 1 mit der in SEQ ID Nr. 1, SEQ ID Nr. 2, SEQ ID Nr. 3 oder SEQ ID Nr. 4 angegebenen Aminosäuresequenz.

3. Polypeptid, das als T dargestellt ist, mit der in SEQ ID Nr. 5, SEQ ID Nr. 6, SEQ ID Nr. 7 oder SEQ ID Nr. 8 angegebenen Aminosäuresequenz, wobei T die allgemeine Formel (Xₙ-His-Xₙ-Yₙ-Pro-Hisₙ)₂₋₆₀ aufweist, wobei X aus der Gruppe ausgewählt ist, die aus Gly, Ala, Ser, Thr, Asp, Glu, His, Val und Leu besteht; Y aus der Gruppe ausgewählt ist, die aus Gly, Ser, Glu, Asp, Lys, Val, Arg, Leu und Thr besteht und n in einem Bereich von 1 bis 4 liegt.

4. Polynukleotidsequenz mit Kodierung für das Polypeptid gemäß Anspruch 1 oder 2, wobei bei Kodierung für das Polypeptid gemäß Anspruch 2 die Nukleotidsequenz die in SEQ ID Nr. 9, SEQ ID Nr. 10, SEQ ID Nr. 11 oder SEQ ID Nr. 12 angegebene ist.

5. Polynukleotidsequenz mit Kodierung für ein als T dargestelltes Polypeptid gemäß Anspruch 3, wobei die Nukleotidsequenz die in SEQ ID Nr. 13, SEQ ID Nr. 14, SEQ ID Nr. 15 oder SEQ ID Nr. 16 angegebene ist.

6. Rekombinanter Expressionsvektor, der einen Promoter, eine in Anspruch 4 beanspruchte Polynukleotidsequenz, eine Proteaseerkennungsstelle oder eine chemische Spaltungsstelle und eine interessierende heterologe DNA umfasst.

7. Rekombinanter Expressionsvektor nach Anspruch 6, wobei der Promoter aus der Gruppe ausgewählt ist, die aus AraB, trp, tac, lac, osmB, CMV, EF-1α, SV 40 und T7 besteht.

8. Rekombinanter Expressionsvektor nach Anspruch 6, wobei die Proteaseerkennungsstelle durch Protease erkannt wird, die aus der Gruppe ausgewählt ist, die aus Enterokinase, Carboxypeptidase, Faktor Xa, Trypsin, V8 und Chymotrypsin, vorzugsweise Enterokinase besteht.

9. Rekombinanter Expressionsvektor nach Anspruch 6, wobei die chemische Spaltungsstelle durch ein Reagenz erkannt wird, das aus der Gruppe ausgewählt ist, die aus Chlorcyan, Hydroxylamin, Ameisensäure, Essigsäure, Salzsäure und Trifluoressigsäure besteht.

10. Rekombinanter Expressionsvektor nach Anspruch 6, wobei die interessierende heterologe DNA aus der Gruppe ausgewählt ist, die aus DNA mit Kodierung für β-Galactosidase, Granulozytenkoloniestimulationsfaktor, humanes Interleukin, Interleukin-2, Thrombozytenwachstumsfaktor, Granulozytenmakrophagenkoloniestimulationsfaktor, Insuline, Rinderenterokinase, Gefäßendothelwachstumsfaktor, Nervenwachstumsfaktor, Interferone und Gewebeplasminogenaktivatoren besteht.

11. Wirtszelle, die den rekombinanten Expressionsvektor nach Anspruch 6 umfasst, vorzugsweise eine Wirtszelle mit der Hinterlegungsnummer MTCC 5312.

12. Wirtszelle nach Anspruch 11, wobei die Wirtszelle aus der Gruppe ausgewählt ist, die aus E.coli, Pseudomonas, Hefe, Saccharomyces, Pichia, Hanseneula, CHO, BHK und COS besteht, wobei es sich vorzugsweise bei der Wirtszelle um E.coli handelt, stärker bevorzugt um E.coli, das aus der Gruppe ausgewählt ist, die aus JM109, DH5α, Top10, BL21, HB101, XL1-Blue und LMG19 besteht.

13. Verfahren zur Herstellung und Reinigung eines rekombinanten Proteins, wobei das Verfahren die folgenden Stufen umfasst: Erhalten und Züchten von rekombinanten Zellen, die den rekombinanten Expressionsvektor nach Anspruch 6 aufweisen, in einem geeigneten Medium zur Herstellung des rekombinanten Proteins; Gewinnen des rekombinanten Proteins durch ein einstufiges Verfahren.

14. Verfahren nach Anspruch 13, wobei das rekombinante Protein aus der Gruppe ausgewählt ist, die aus β-Galactosidase, Granulozytenkoloniestimulationsfaktor, humanes Interleukin, Interleukin-2, Thrombozytenwachstumsfaktor, Granulozytenmakrophagenkoloniestimulationsfaktor, Insulinen, Rinderenterokinase, Gefäßendothelwachstumsfaktor, Nervenwachstumsfaktor, Interferonen und Gewebeplasminogenaktivatoren besteht.

15. Verfahren nach Anspruch 13, wobei die Gewinnung des rekombinanten Proteins durch Reinigung unter Verwendung einer Chelatbildungschromatographie mit immobilisierten Metallionen erfolgt.

16. Verfahren nach Anspruch 15, wobei die Chelatbildungschromatographie mit immobilisierten Metallionen Metallionen verwendet, die aus der Gruppe ausgewählt sind, die aus Ni, Cu, Zn und Co besteht.

## Revendications

1. Polypeptide d'affinité ayant la formule générale R.sup.1-T-R.sup.2, où R.sup.1 est un peptide comprenant 1-30 acide(s) aminé(s) ; T ayant la formule générale (Xₙ-His-Xₙ-Yₙ-Pro-HiSₙ)₂₋₆₀, dans laquelle X est sélectionné dans un groupe consistant en Gly, Ala, Ser, Thr, Asp, Glu, His, Val, et Leu ; Y est sélectionné dans le groupe consistant en Gly, Ser, Glu, Asp, Lys, Val, Arg, Leu et Thr ; n est compris entre 1 et 4 ; et R.sup.2 est Z, ou Z-Asp-Asp-Asp-Asp-Lys- ou Z-Ile-Glu-Gly-Arg-, où Z est un peptide comprenant entre 1 et 30 acide(s) aminé(s).

2. Polypeptide selon la revendication 1, ayant la séquence d'acides aminés telle que montrée dans SEQ ID NO: 1, SEQ ID NO: 2, SEQ ID NO: 3 ou SEQ ID NO: 4.

3. Polypeptide représenté par T ayant une séquence d'acides aminés telle que montrée dans SEQ ID NO: 5, SEQ ID NO: 6, SEQ ID NO: 7 ou SEQ ID NO: 8, où T possède une formule générale (Xₙ-His-Xₙ-Yₙ-Pro-Hisₙ)₂₋₆₀, dans laquelle X est sélectionné dans un groupe consistant en Gly, Ala, Ser, Thr, Asp, Glu, His, Val, et Leu ; Y est sélectionné dans le groupe consistant en Gly, Ser, Glu, Asp, Lys, Val, Arg, Leu et Thr ; n est compris entre 1 et 4.

4. Séquence polynucléotidique codant pour le polypeptide selon la revendication 1 ou 2, où, si elle code pour le polypeptide selon la revendication 2, la séquence de nucléotides est telle que montrée dans SEQ ID NO: 9, SEQ ID NO: 10, SEQ ID NO: 11 ou SEQ ID NO: 12.

5. Séquence polynucléotidique codant pour le polypeptide représenté par T selon la revendication 3, où la séquence de nucléotides est telle que montrée dans SEQ ID NO: 13, SEQ ID NO: 14, SEQ ID NO: 15 ou SEQ ID NO: 16.

6. Vecteur d'expression recombinant comprenant un promoteur, une séquence polynucléotidique selon la revendication 4, un site de reconnaissance de protéase ou un site de clivage chimique et un ADN hétérologue d'intérêt.

7. Vecteur d'expression recombinant selon la revendication 6, dans lequel ledit promoteur est sélectionné dans un groupe consistant en AraB, trp, tac, lac, osmB, CMV, EF-1α, SV 40 et T7.

8. Vecteur d'expression recombinant selon la revendication 6, dans lequel le site de reconnaissance de protéase est reconnu par une protéase sélectionnée dans le groupe consistant en une entérokinase, une carboxypeptidase, le facteur Xa, la trypsine, V8, et la chymotrypsine, de préférence une entérokinase.

9. Vecteur d'expression recombinant selon la revendication 6, dans lequel ledit site de clivage chimique est reconnu par un réactif sélectionné dans un groupe consistant en un halogénure de cyanogène, une hydroxylamine, l'acide formique, l'acide acétique, l'acide chlorhydrique et l'acide trifluoroacétique.

10. Vecteur d'expression recombinant selon la revendication 6, dans lequel ledit ADN hétérologue d'intérêt est sélectionné dans un groupe consistant en un ADN codant pour la β-galactosidase, le facteur de stimulation des colonies de granulocytes, une interleukine humaine, l'interleukine-2, le facteur de croissance dérivé des plaquettes, le facteur de stimulation des colonies de granulocytes-macrophages, des insulines, une entérokinase bovine, le facteur de croissance de l'endothélium vasculaire, le facteur de croissance des nerfs, des interférons et des activateurs tissulaires du plasminogène.

11. Cellule hôte comprenant le vecteur d'expression recombinant selon la revendication 6, de préférence une cellule hôte ayant le numéro d'accès MTCC 5312.

12. Cellule hôte selon la revendication 11, où ladite cellule hôte est sélectionnée dans un groupe consistant en E.coli, Pseudomonas, une levure, Saccharomyces, Pichia, Hansenula, CHO, BHK et COS, la cellule hôte étant de préférence E.coli, ladite E.coli étant de manière davantage préférée sélectionnée dans un groupe consistant en JM 109, DH5α, Top10, BL21, HB101, XLI-Blue et LMG19.

13. Procédé pour la production et la purification d'une protéine recombinante, ledit procédé consistant à obtenir et à cultiver des cellules recombinantes comportant le vecteur d'expression recombinant selon la revendication 6, dans un milieu approprié pour la production de la protéine recombinante ; et à récupérer ladite protéine recombinante par un procédé en une seule étape.

14. Procédé selon la revendication 13, dans lequel ladite protéine recombinante est sélectionnée dans un groupe consistant en la β-galactosidase, le facteur de stimulation des colonies de granulocytes, une interleukine humaine, l'interleukine-2, le facteur de croissance dérivé des plaquettes, le facteur de stimulation des colonies de granulocytes-macrophages, des insulines, une entérokinase bovine, le facteur de croissance de l'endothélium vasculaire, le facteur de croissance des nerfs, des interférons et des activateurs tissulaires du plasminogène.

15. Procédé selon la revendication 13, dans lequel ladite récupération de la protéine recombinante est réalisée par une purification en utilisant une chromatographie par chélation d'ions métalliques immobilisés.

16. Procédé selon la revendication 15, dans lequel ladite chromatographie par chélation d'ions métalliques immobilisés emploie un ion métallique sélectionné dans un groupe consistant en Ni, Cu, Zn et Co.


### SEQUENCE LISTING

<110> USV LIMITED
   Rao, Laxmi S
   Mishra , Shrikant
   Borbhuiya, Monsur Ahmed
   Mhatre, Deepa
   Thakur, Priti
<120> AFFINITY POLYPEPTIDE FOR PURIFICATION OF RECOMBINANT PROTEINS
<130> IPA0199
<160> 33
<170> PatentIn version 3.3
<210> 1
   <211> 48
   <212> PRT
   <213> ARTIFICIAL
<220>
   <223> SYNTHETIC SEQUENCE
<400> 1
   Met Ala Leu His Ala His Leu Asp Pro His Leu Val Thr Glu His Ala
   1               5                   10                  15
   His Leu Asp Pro His Ala Ser Leu His Ala His Leu Asp Pro His Leu
               20                  25                  30
   Val Thr Glu His Ala His Leu Asp Pro His Val Asp Asp Asp Asp Lys
           35                  40                  45
<210> 2
   <211> 49
   <212> PRT
   <213> ARTIFICIAL
<220>
   <223> SYNTHETIC SEQUENCE
<400> 2
   Met Val Ala His Leu His Ala Ser Pro His Ala Ala Asp Thr His Val
   1               5                   10                  15
   His Ala Ser Pro His Val Thr Ala His Val His Ala Ser Pro His Ala
               20                  25                  30
   Ala Asp Thr His Val His Ala Ser Pro His Leu Val Asp Asp Asp Asp
           35                  40                  45
   Lys
<210> 3
   <211> 50
   <212> PRT
   <213> ARTIFICIAL
<220>
   <223> SYNTHETIC SEQUENCE
<400> 3
   Met Leu Val His Leu His Val Glu Pro His Val Leu Ser Asp His Leu
   1               5                   10                  15
   His Val Glu Pro His Leu Asp Val His Leu His Val Glu Pro His Val
               20                  25                  30
   Leu Ser Asp His Leu His Val Glu Pro His Leu Ser Val Asp Asp Asp
           35                  40                  45
   Asp Lys
       50
<210> 4
   <211> 52
   <212> PRT
   <213> ARTIFICIAL
<220>
   <223> SYNTHETIC SEQUENCE
<400> 4
   Met Gly Leu His Val His Gly Thr Pro His Gly Leu Glu Ser His Gly
   1               5                   10                  15
   His Gly Thr Pro His Gly Glu Gly His Leu His Gly Thr Pro His Gly
               20                  25                  30
   Leu Glu Ser His Gly His Val Thr Pro His Gly Val Ala Ser Leu Asp
           35                  40                  45
   Asp Asp Asp Lys
       50
<210> 5
   <211> 41
   <212> PRT
   <213> ARTIFICIAL
<220>
   <223> SYNTHETIC SEQUENCE
<400> 5
   Ala Leu His Ala His Leu Asp Pro His Leu Val Thr Glu His Ala His
   1               5                   10                  15
   Leu Asp Pro His Ala Ser Leu His Ala His Leu Asp Pro His Leu Val
               20                  25                  30
   Thr Glu His Ala His Leu Asp Pro His
           35                  40
<210> 6
   <211> 41
   <212> PRT
   <213> ARTIFICIAL
<220>
   <223> SYNTHETIC SEQUENCE
<400> 6
   Val Ala His Leu His Ala Ser Pro His Ala Ala Asp Thr His Val His
   1               5                   10                  15
   Ala Ser Pro His Val Thr Ala His Val His Ala Ser Pro His Ala Ala
               20                  25                  30
   Asp Thr His Val His Ala Ser Pro His
           35                  40
<210> 7
   <211> 41
   <212> PRT
   <213> ARTIFICIAL
<220>
   <223> SYNTHETIC SEQUENCE
<400> 7
   Leu Val His Leu His Val Glu Pro His Val Leu Ser Asp His Leu His
   1               5                   10                  15
   Val Glu Pro His Leu Asp Val His Leu His Val Glu Pro His Val Leu
               20                  25                  30
   Ser Asp His Leu His Val Glu Pro His
           35                  40
<210> 8
   <211> 41
   <212> PRT
   <213> ARTIFICIAL
<220>
   <223> SYNTHETIC SEQUENCE
<400> 8
   Gly Leu His Val His Gly Thr Pro His Gly Leu Glu Ser His Gly His
   1               5                   10                  15
   Gly Thr Pro His Gly Glu Gly His Leu His Gly Thr Pro His Gly Leu
               20                  25                  30
   Glu Ser His Gly His Val Thr Pro His
           35                  40
<210> 9
   <211> 144
   <212> DNA
   <213> ARTIFICIAL
<220>
   <223> SYNTHETIC SEQUENCE
<400> 9
   atggcactgc acgcacatct ggaccctcat ctggtgacgg agcacgccca cctcgatccg 60
   cacgctagcc tgcacgcaca tctggaccct catctggtga cggaacacgc ccacctcgat 120
   ccgcacgtcg acgacgacga caag 144
<210> 10
   <211> 147
   <212> DNA
   <213> ARTIFICIAL
<220>
   <223> SYNTHETIC SEQUENCE
<400> 10
   atggtggcac atctgcatgc aagcccgcat gcagcagata cccatgttca tgcaagcccg 60
   catgtgaccg cacatgttca tgcaagcccg catgcagcag atacccatgt tcatgcaagc 120
   ccgcatttag tcgacgacga cgacaag 147
<210> 11
   <211> 150
   <212> DNA
   <213> ARTIFICIAL
<220>
   <223> SYNTHETIC SEQUENCE
<400> 11
   atgctggtgc atctgcatgt tgaaccgcat gttctgagcg atcatctgca tgttgaaccg 60
   catctggatg tgcatctgca tgtggaaccg catgtgctga gcgatcatct gcatgtggaa 120
   ccgcatctgt ctgttgacga cgacgacaag 150
<210> 12
   <211> 155
   <212> DNA
   <213> ARTIFICIAL
<220>
   <223> SYNTHETIC SEQUENCE
<400> 12
   atgggtctgc atgtgcatgg taccccgcat ggtctggaaa gccatggtca tggtaccccg 60
   catggtgaag gtcatctgat ggtaccccgc atggtctgga aagccatggt catgtgaccc 120
   cgcatggtgt tgcaagctta gacgacgacg acaag 155
<210> 13
   <211> 123
   <212> DNA
   <213> ARTIFICIAL
<220>
   <223> SYNTHETIC SEQUENCE
<400> 13
   gcactgcacg cacatctgga ccctcatctg gtgacggagc acgcccacct cgatccgcac 60
   gctagcctgc acgcacatct ggaccctcat ctggtgacgg aacacgccca cctcgatccg 120
   cac 123
<210> 14
   <211> 123
   <212> DNA
   <213> ARTIFICIAL
<220>
   <223> SYNTHETIC SEQUENCE
<400> 14
   gtggcacatc tgcatgcaag cccgcatgca gcagataccc atgttcatgc aagcccgcat 60
   gtgaccgcac atgttcatgc aagcccgcat gcagcagata cccatgttca tgcaagcccg 120
   cat 123
<210> 15
   <211> 123
   <212> DNA
   <213> ARTIFICIAL
<220>
   <223> SYNTHETIC SEQUENCE
<400> 15
   ctggtgcatc tgcatgttga accgcatgtt ctgagcgatc atctgcatgt tgaaccgcat 60
   ctggatgtgc atctgcatgt ggaaccgcat gtgctgagcg atcatctgca tgtggaaccg 120
   cat 123
<210> 16
   <211> 122
   <212> DNA
   <213> ARTIFICIAL
<220>
   <223> SYNTHETIC SEQUENCE
<400> 16
   ggtctgcatg tgcatggtac cccgcatggt ctggaaagcc atggtcatgg taccccgcat 60
   ggtgaaggtc atctgatggt accccgcatg gtctggaaag ccatggtcat gtgaccccgc 120
   at 122
<210> 17
   <211> 29
   <212> DNA
   <213> ARTIFICIAL
<220>
   <223> SYNTHETIC SEQUENCE
<400> 17
   ggtgtcggcg atttttaacc gtgactatc 29
<210> 18
   <211> 22
   <212> DNA
   <213> ARTIFICIAL
<220>
   <223> SYNTHETIC SEQUENCE
<400> 18
   ctgccgctac cgccgactaa cg 22
<210> 19
   <211> 36
   <212> DNA
   <213> ARTIFICIAL
<220>
   <223> SYNTHETIC SEQUENCE
<400> 19
   ctggcgctga ccggtatcgc cctgctggcg ctgatt 36
<210> 20
   <211> 42
   <212> DNA
   <213> ARTIFICIAL
<220>
   <223> SYNTHETIC SEQUENCE
<400> 20
   cgtagttgtc gacatgaggg tccaggtgtg cgtgctccgt ca 42
<210> 21
   <211> 37
   <212> DNA
   <213> ARTIFICIAL
<220>
   <223> SYNTHETIC SEQUENCE
<400> 21
   ggtggccatg gcactgcacg cacatctgga ccctcat 37
<210> 22
   <211> 31
   <212> DNA
   <213> ARTIFICIAL
<220>
   <223> SYNTHETIC SEQUENCE
<400> 22
   gttcccaacg atcaaggcga gttacatgat c 31
<210> 23
   <211> 30
   <212> DNA
   <213> ARTIFICIAL
<220>
   <223> SYNTHETIC SEQUENCE
<400> 23
   gctaaccaaa ccggtaaccc cgcttattaa 30
<210> 24
   <211> 34
   <212> DNA
   <213> ARTIFICIAL
<220>
   <223> SYNTHETIC SEQUENCE
<400> 24
   ggcgtgctcc gtcaccagat gagggtccag atgt 34
<210> 25
   <211> 41
   <212> DNA
   <213> ARTIFICIAL
<220>
   <223> SYNTHETIC SEQUENCE
<400> 25
   ctggtgacgg agcacgccca cctcgatccg cacgtcgacg a 41
<210> 26
   <211> 39
   <212> DNA
   <213> ARTIFICIAL
<220>
   <223> SYNTHETIC SEQUENCE
<400> 26
   ggataatacc gcgccacata gcagaacttt aaaagtgct 39
<210> 27
   <211> 30
   <212> DNA
   <213> ARTIFICIAL
<220>
   <223> SYNTHETIC SEQUENCE
<400> 27
   ctaaccaaac cggtaacccc gcttattaaa 30
<210> 28
   <211> 31
   <212> DNA
   <213> ARTIFICIAL
<220>
   <223> SYNTHETIC SEQUENCE
<400> 28
   ccagtctttc gactgagcct ttcgttttat t 31
<210> 29
   <211> 27
   <212> DNA
   <213> ARTIFICIAL
<220>
   <223> SYNTHETIC SEQUENCE
<400> 29
   gctcttctcg ctaaccaaac cggtaac 27
<210> 30
   <211> 27
   <212> DNA
   <213> ARTIFICIAL
<220>
   <223> SYNTHETIC SEQUENCE
<400> 30
   cgtcgtcgct agcgtgcgga tcgaggt 27
<210> 31
   <211> 36
   <212> DNA
   <213> ARTIFICIAL
<220>
   <223> SYNTHETIC SEQUENCE
<400> 31
   ggaggaatta accgctagcc tgcacgcaca tctgga 36
<210> 32
   <211> 25
   <212> DNA
   <213> ARTIFICIAL
<220>
   <223> SYNTHETIC SEQUENCE
<400> 32
   gcagcaacga gacgtcacgg aaaat 25
<210> 33
   <211> 204
   <212> DNA
   <213> ARTIFICIAL
<220>
   <223> SYNTHETIC SEQUENCE
<400> 33
   atggcactgc acgcacatct ggaccctcat ctggtgacgg agcacgccca cctcgatccg     60
   cacgctagcc tgcacgcaca tctggaccct catctggtga cggaacacgc ccacctcgat    120
   ccgcacgtcg acgacgacga caaggatcca atgatagatc ccgtcgtttt acaacgtcgt    180
   gactgggaaa accctggcgt tacc                                           204